# EUROPEAN PATENT SPECIFICATION

(11) **EP 3 717 025 B1**
(45) Date of publication and mention of the grant of the patent: **03.11.2021**
(21) Application number: 18811010.0
(22) Date of filing: 27.11.2018
(51) Int. Cl.: A61K 51/04, C07B 59/00, C07D 491/048, A61K 101/00, A61K 101/02

(54) **IMAGING AGENTS**
KONTRASTMITTEL
AGENTS D'IMAGERIE

(30) Priority: 01.12.2017 EP 17204896
(43) Date of publication of application: 07.10.2020
(73) Proprietor: UCB Biopharma SRL, 1070 Brussels (BE)
(72) Inventor: MERCIER, Joël, 1070 Brussels (BE); VALADE, Anne, 1070 Brussels (BE); VERMEIREN, Celine, 1070 Brussels (BE); WOOD, Martyn, Brussels 1070 (BE); MAGUIRE, Ralph, 1070 Brussels (BE)
(74) Representative: UCB Intellectual Property
(86) International application number: PCT/EP2018/082649
(87) International publication number: WO 2019/105913

(56) References cited:
- WO-A1-2014/072881

## Description

### FIELD OF THE INVENTION

The present invention relates to radiolabelled 4-(furo[3,2-c]pyridin-4-yl) derivatives and their use as radioactive tracers, and in particular as imaging agents.

In particular, the present invention relates to radiolabelled 6-[2-(fluoromethyl)-4-(furo[3,2-c]pyridin-4-yloxy)phenyl]-1,5-dimethylpyrimidine-2,4(1H,3H)-dione compounds, and their use as radioactive tracers and in particular as imaging agents.

More particularly, the invention relates to the use of radiolabelled 6-[2-(fluoromethyl)-4-(furo[3,2-c]pyridin-4-yloxy)phenyl]-1,5-dimethylpyrimidine-2,4(1H,3H)-dione compounds as PET imaging agents.

### BACKGROUND OF THE INVENTION

Dopaminergic neurons in human brain play a central role in brain health and function. Dopaminergic neurotransmission is important for movement, cognition and reward behavior. Five post-synaptic dopaminergic neuroreceptors have been identified (D₁-D₅).

The monoamine dopamine acts via two families of GPCRs to modulate motor function, reward mechanisms, cognitive processes and other physiological functions. Specifically, dopamine acts upon neurons via D1-like, comprising dopamine D1 and D5, receptors which couple mainly to the Gₛ G-protein and thereby stimulate cAMP production, and D2-like, which comprise D2, D3 and D4, receptors which couple to G_{i/q} G-proteins and which attenuate cAMP production. These receptors are widely expressed in different brain regions.

Medicines based on antagonist and agonist modes of action at dopaminergic receptors have been developed for Parkinson's disease, schizophrenia restless leg's syndrome and ADHD as reported in Stocchi, F. et al. Advances in dopamine receptor agonists for the treatment of Parkinson's disease. Expert Opin Pharmacother 2016, 17(14), 1889-1902; Buckley, P. F. Broad therapeutic uses of atypical antipsychotic medications. Biol Psychiatry 2001, 50(11), 912-924; and Davidson, M. A. ADHD in adults: a review of the literature. J Atten Disord 2008, 11(6), 628-641.

For some GPCRs, however, it has proven challenging to develop small molecules or to achieve sufficient selectivity due to the high degree of homology in the ligand binding site between subtypes, e.g. dopamine D1 and D5 (D1-like) or D2 and D3, and therefore existing medicines are not completely devoid of side effects.

Despite many efforts over the last 15 years to decrease the attrition in drug development, there is still less than a 10% chance that a drug entering clinical trials will reach the market.

Attrition is highest for oncology and CNS drug development, in particular with novel targets with limited understanding of the biology. In order to reduce drug attrition in clinical trials, there is a clear need to develop reliable translational evaluation tools that will enable faster and better decisions, as illustrated in Woodcock, J. and al. The FDA critical path initiative and its influence on new drug development. Annu Rev Med 2008, 59, 1-12.

Because accessing brain tissues in living human is not possible, the development of such translational tools to support CNS drug discovery has to rely inter alia on non-invasive techniques like *in vivo* imaging. Over the last years, the use of positron emission tomography (PET) imaging has extensively developed to support CNS drug discovery.

PET is a precise and sophisticated technique relying on isotopes produced in a cyclotron. A positron-emitting radionuclide is introduced, e.g. by injection, and accumulates in the target tissue. As it decays it emits a positron which promptly combines with a nearby electron resulting in simultaneous emission of two identifiable gamma rays in opposite directions. They are detected by a PET camera and give a precise indication of their origin. PET is a very sensitive technique and therefore requires a small quantity of radiolabeled compounds which are called PET tracers or PET imaging agents or PET ligands.

PET imaging is particularly well suited as it relies on the incorporation of radioisotopes of atoms that are present in most small molecule drug candidates. It can provide quantitative information to support the decision-making process at different stages of preclinical and clinical drug development.

For example, PET imaging can provide brain pharmacokinetic information in living subjects. Through the direct radiolabelling of a drug candidate, information regarding the blood-brain barrier (BBB) penetration in both healthy and diseased subjects can be obtained.

The development of a validated PET imaging agent for the protein/receptor targeted by the drug development program also provides the opportunity to assess, through blocking studies, the target occupancy or engagement of a drug candidate. These studies may provide critical information such as the relationship between target occupancy and administered dose, as well as the target occupancy kinetics. When obtained in phase I of clinical development, such information may be decision-making to select the most appropriate dose range and regimen for phase II POC studies.

PET imaging studies can also provide some insight into the mechanism of action of a drug candidate and can allow patient stratification, monitoring of disease progression, or be used for proof of pharmacology or as objective endpoints in Phase II POC studies.

Designing PET imaging agents requires compounds with specific properties that are not necessarily aligned with the properties of drug candidates.

Ideal properties required for compounds to be suitable as PET imaging agents have been reported in Zhang, L et al. Strategies to facilitate the discovery of novel CNS PET ligands. EJNMMI Radiopharmacy and Chemistry 2016, 1 (13), 1-12; Need, A et al. Approaches for the discovery of novel positron emission tomography radiotracers for brain imaging. Clin. Transl. Imaging 2017, 5 (3), 265-274; Pike, V. W. Considerations in the development of reversibly binding PET radioligands for brain imaging. Curr Med Chem. 2016, 23 (18), 1818-1869; Zhang, L. et al. Design and selection parameters to accelerate the discovery of novel central nervous system PET ligands and their application in the development of a novel phosphodiesterase 2A PET ligand. J Med Chem. 2013, 56 (11), 4568-4579. However, appropriate combination of these properties are often complicated to achieve.

Furthermore, beyond *in vivo* imaging, radioactive tracers that can be used *in vitro or ex vivo,* e.g. in autoradiography of animal tissues, are equally important in drug development decision making. These studies help to understand the mechanism of action of compounds modulating a particular receptor linked to one or more diseases, or to understand changes in target density resulting from compound pharmacological action.

D1-like receptors are involved in numerous physiological functions and behavioural processes. For example, they are involved in synaptic plasticity, cognitive function and goal-directed motor functions, but also in reward processes. Due to their role in several physiological/neurological processes, D1-like receptors have been implicated in a variety of disorders including cognitive and negative symptoms in schizophrenia, cognitive impairment related to classical antipsychotic therapy, impulsivity, attention disorder with hyperactivity (ADHD), Parkinson's disease and related movement disorders, dystonia, Huntington's disease, dementia with Lewy Body, Alzheimer's disease, age-related cognitive decline, mild cognitive impairment (MCI), drug addiction sleep disorders, and apathy.

Some PET imaging agents have been developed for dopaminergic neuroreceptor subtypes, as reported in Prante, O., et al. Radioligands for the dopamine receptor subtypes. J Labelled Comp Radiopharm 2013, 56(3-4), 130-148.

However these PET imaging agents present certain drawbacks such as lack of selectivity for the D1-like receptors, in particular in the neocortex of the brain, non suitable pharmacokinetic properties or generation of radioactive metabolites which penetrate the brain blood barrier and may impair quantitification measurements.

Most of these PET imaging agents are also based on antagonists rather than agonists.

It is therefore desirable to develop orthosteric agonist imaging agents of D1-like receptors, in particular PET imaging agents, because they would be more sensitive to orthosteric D1 modulators, as well as to the levels of dopamine, the endogenous D1 ligand. This could allow more precise study on the effect of drugs targeting that receptor site and to determine changes in receptor stimulation in disease states.

In recent years, there has been a renewed interest to develop non-catechol selective D1-like orthosteric agonists or allosteric modulators which modulate the activity of dopamine receptors, in particular D1 receptor, and that could be used to treat D1 mediated disorders.

D1 non-catechol orthosteric ligands are, for example, described in international patent application published under n°WO 2014/072881.

D1-like allosteric modulators are, for example, described in international patent applications published under n°WO 2014/193781 and n°WO2016/055479.

Given this renewed interest, there is a need for selective D1-like imaging agents, in particular D1-like PET imaging agents which could be used in *in vitro*, *ex vivo* or *in vivo* testing and also in human clinical studies, either to support the development of drug candidates or to delineate D1 receptor roles in pathologies of interest.

### SUMMARY OF THE INVENTION

One objective of the present invention is therefore to provide compounds which are selective agonists of D1-like receptor and that are useful as radioactive tracers, in particular as PET imaging agents.

It is also an objective of the present invention to provide a method of imaging and quantifying the expression of the D1 receptor using the compounds according to the present invention.

Another objective of the present invention is to provide methods for *in vitro* or *ex vivo* detection and quantification of the density of available D1-like receptor in brain tissues, with or without an endogenous or exogenous ligand, which method comprises treating the tissue with a radiolabeled compound according to the present invention and measuring the binding level of said radiolabeled compound.

Another objective of the present invention is to provide methods for *in vivo* detection and quantification of the density of available D1-like receptor in brain, with or without an endogenous or exogenous ligand, which method comprises administering to the subject an effective amount of a radiolabeled compound according to the present invention and measuring the brain uptake level of said radiolabeled compound in a relevant region of interest.

A further objective of the present invention is to provide a method for *in vitro* or *ex vivo* detection and quantification of the effect of a D1 PAM on the availability of the D1 receptor in brain tissues, which method comprises treating the tissue with a radiolabeled compound according to the present invention in combination with a D1 PAM and detecting the increased binding of said radiolabeled compound in presence of the PAM.

Yet another objective of the present invention is to provide a method for *in vivo* detection and quantification of the effect of a D1 PAM on the availability of the D1 receptor in the brain of a subject, which method comprises administering to the subject an effective amount of a radiolabeled compound according to the present invention, in combination with administering a D1 PAM and detecting the increased brain uptake of said radiolabeled compound in a relevant region of interest compared to baseline conditions.

Further aspects of the invention will become apparent from the detailed description.

### DESCRIPTION OF THE DRAWINGS

Figure 1 shows the result of a cell-based D1 receptor functional assay with a compound of formula (I), as described here below.
Figure 2 shows brain *in vivo* PET imaging in monkey using a radiolabeled compound of formula (I) as described here below.
Figure 3 shows *in vitro* binding experiments with a radiolabeled compound of formula (I) as described here below, and radiolabeled D1 antagonist to human recombinant D1 receptor in the absence or the presence of a D1 PAM.
Figure 4 shows autoradiography *ex vivo* binding experiments with a radiolabeled compound of formula (I) in non-human primate (NHP) brain tissues, in the absence or the presence of a D1 PAM.
Figure 5 shows *in vivo* brain PET imaging performed in monkeys in the presence of a radiolabeled compound of formula (I) with or without a D1 PAM, and associated Standardized uptake value (SUV) time-activity curves.
Figure 6 is a potentiation plot showing the increased volume of distribution (V_{T}) of a radiolabeled compound of formula (I) in different regions of the brain post in vivo dosing with a D1 PAM.

### DETAILED DESCRIPTION OF THE INVENTION

The present invention relates to a compound of formula (I), which is radiolabeled, or pharmaceutically acceptable salt thereof,

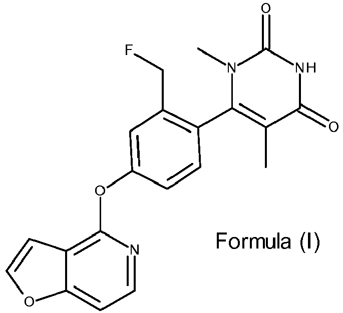

In particular, the present invention relates to a compound of formula (I) or a pharmaceutical acceptable salt thereof, wherein at least one of the hydrogen is ³H; or at least one of the carbons is a ¹¹C; or the fluorine atom is a ¹⁸F.

In one aspect, the present invention relates to a compound of formula (I) or a pharmaceutically acceptable salt thereof, wherein one of the carbon is a ¹¹C, herein after referred to as compound of formula (Ia).

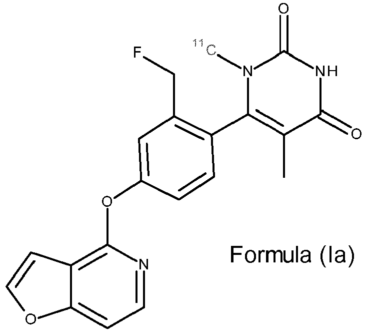

In another aspect, the present invention relates to a compound of formula (I) or a pharmaceutically acceptable salt thereof, wherein the flurorine is a ¹⁸F, herein after referred to as compound of formula (lb).

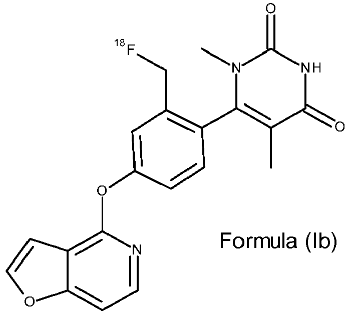

In a further aspect, the present invention relates to a compound of formula (I) or a pharmaceutically acceptable salt thereof, wherein one or more of the hydrogens is ³H, herein after referred to as compound of formula (Ic),

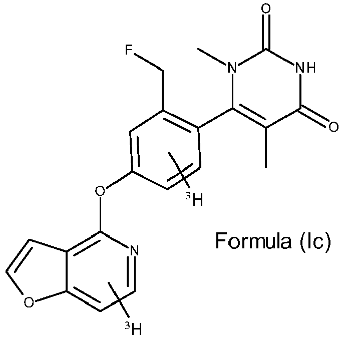

The term "radiolabeled" as used herein with reference to a particular compound means labeled with a radioactive isotope.

A radioactive isotope is any of several species of a chemical element with different masses whose nuclei are unstable and dissipate excess energy by spontaneously emitting radiation in the form of alpha, beta, and gamma rays. Specific examples of radioactive isotopes according to the present invention are ³H, ¹⁸F, and ¹¹C.

The present invention includes within its scope pharmaceutically acceptable salts of the compounds of formula (I) above. For use in medicine, the salts of the compounds of formula (I) will be pharmaceutically acceptable salts. Other salts may, however, be useful in the preparation of the compounds of use in the invention or of their pharmaceutically acceptable salts. Standard principles underlying the selection and preparation of pharmaceutically acceptable salts are described, for example, in Handbook of Pharmaceutical Salts: Properties, Selection and Use, ed. P.H. Stahl & C.G. Wermuth, Wiley-VCH, 2002.

With respect to the present invention, reference to a compound or compounds is intended to encompass that compound in each of its possible isomeric forms and mixtures thereof, unless the particular isomeric form is referred to specifically.

In particular, reference to compound or compounds is intended to encompass that compound in each of its possible atropoisomers.

Atroposiomers are stereoisomers arising because of hindered rotation about a single bond, where energy differences due to steric strain or other contributors create a barrier to rotation that is high enough to allow for isolation of individual conformers (see for example Bringmann G. et al. Atroposelective Synthesis of Axially Chiral Biaryl Compounds. Angewandte Chemie International Edition. (2005) 44 (34): 5384-5427)

Atropoisomers are generally identified as isomer (+) and (-) and their configuration is determined by measuring the optical rotation [α]_{D} of the compound in a polarimeter according to methods well-known to the skilled in the art.

In one embodiment, the present invention relates to radiolabeled compound of formula (I) which is a 50:50 mixture of atropisomers (+) and (-). In a particular embodiment the present invention relates to radiolabeled compound of formula (I) which consist essentially of atropisomer (-).

The term "consisting essentially" by reference to an atropoisomer means that the atropisomer is present in a ratio of at least 95:5 with respect to the other atropoisomer.

Radiolabeled compounds of formula (I) according to the present invention are orthosteric agonists of D1. They are particularly advantangeous because they are selective for the D1-like receptors compared to the existing radiolabelled D1 tracers which also bind to the serotonin 5-HT2A receptor.

Hence in one embodiment, the present invention relates to compounds of formula(I) as herein described which are selective orthosteric agonists of D1-like receptor.

Typically, compounds of formula (I) as described herein have an affinity for the D1-like receptors, expressed in the form of a pKi value, which is at least of about 8, preferably at least of about 9. This means that compounds of formula (I) as described herein have an affinity in the range of low nanomolar for these receptors. Specific values are provided in the experimental section.

It will be understood by the person skilled in the art that values of pKi obtained with non radiolabeled compounds of formula (I) are transposable to the corresponding radiolabeled compounds.

Competitive radioligand binding assays were performed to measure the affinity of compounds of formula (I) for D1 receptor and assess its selectivity regarding a large panel of 80 related and non-related biological targets. It was shown that compounds of formula (I) bind to D1 and D5 receptors (herein after referred to as "D1-like receptors") with an affinity in the subnanomolar range and do not significantly bind to any of the other tested targets.

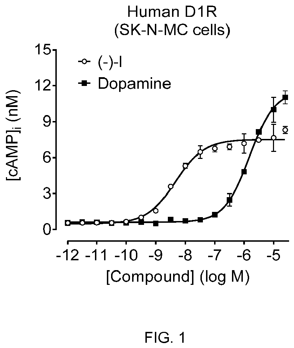

Furthermore, as shown in Figure 1 and as further detailed in the experimental section, activity and potency of compounds of formula (I) in a D1 cell based functional assay were performed and compared with dopamine, the endogenous agonist ligand of D1 receptor. In this assay, it was shown that the D1 receptor was activated *in vitro* by compound of formula (I) likely by the dopamine. Furthermore compounds according to the invention display a better potency (pEC50 value of at least 8) as compared to the endogenous ligand dopamine. Specific values and conditions are further detailed in the experimental section.

Evidence mentioned here above and in the experimental section supports the fact that radiolabeled compounds of formula (I) are selective agonist ligands of D1-like receptors.

This makes radiolabeled compounds of formula (I) as described herein particularly suitable for use in PET imaging studies either alone or in combination with D1 orthosteric or allosteric modulators developed for the treatment of D1 mediated diseases.

In another embodiment, the present invention relates to the use of radiolabeled compounds of formula (I) as herein described for *in vitro* or *ex vivo* studies.

In a particular aspect according to this embodiment, the present invention relates to the use of compounds of formula (Ic) in *in vitro* or *ex vivo* studies.

The present invention also relates to a method of detecting the expression of the D1 receptor in the brain which comprises the *in vitro* or *ex vivo* incubation of a detectable amount of a radiolabeled compound of formula (I), as described herein, and detecting said radiolabeled compound.

Such method allows the quantification of the density of available D1 receptor in diseases in which D1 receptors play a role.

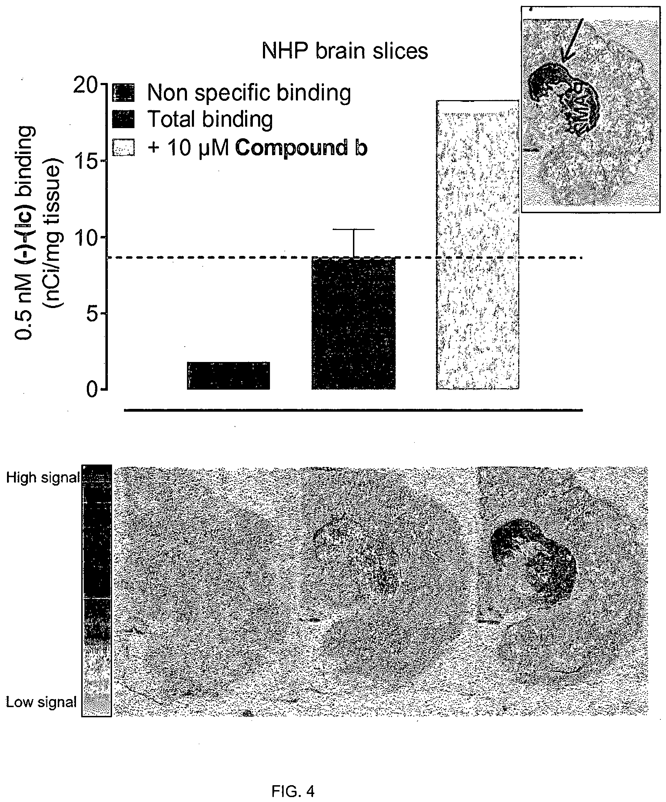

The graph in Figure 4 shows that compound of formula (Ic) significantly binds in caudate putamen consistent with highest levels of D1 receptor expression found in the striatum region of brain tissue.

In another embodiment, the present invention relates to compound of formula (I) for use in *in vivo* PET imaging studies. In one aspect of this embodiment, the present invention relates to compound of formula (Ia) or (Ib) for use in *in vivo* PET imaging studies.

In another embodiment, the present invention relates to compound of formula (I) for use *in vivo* PET imaging.

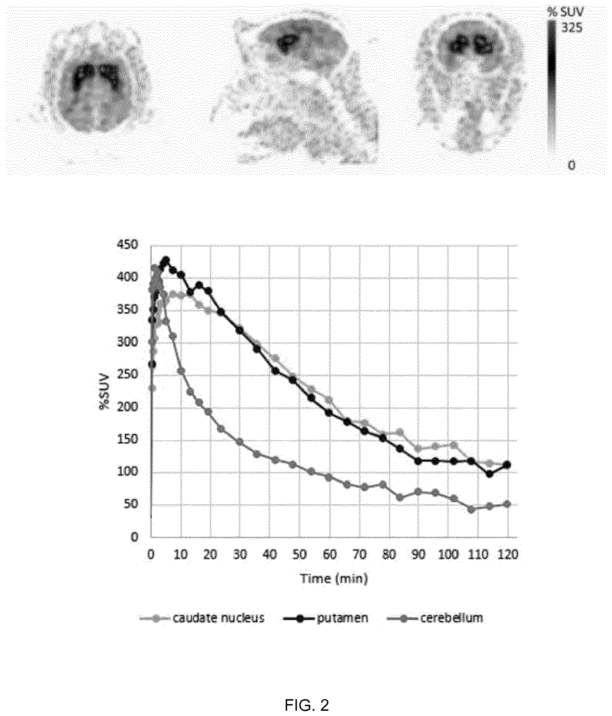

Figure 2 shows PET imaging performed with compound of formula (Ia) in a cynomolgus monkey and which demonstrates an uptake pattern consistent with the reported distribution of D1-like receptors in the brain.

In a particular aspect of this embodiment, the present invention relates to compounds of formula (I) for use in a method of *in vivo* diagnosis of D1 mediated disorders.

In another particular aspect of this embodiment, the present invention relates to compounds of formula (I) as herein described for use in a method of *in vivo* quantification of the density of available D1 receptor in diseases in which D1 receptors play a role.

The present invention also encompasses within its scope a radiolabelled compound of formula (I), as described herein, for use in a method of *in vivo* imaging and detection of neurological disorders mediated by D1 receptor which comprises administering a detectable amount of a radiolabelled compound of formula (I), as described herein, to a subject in need thereof, or its pharmaceutically acceptable salt, and detecting the radiolabelled compound bound to the D1 receptor.

Some *in vivo* studies have been performed with radiolabeled compound of formula (I), and in particular with compound of formula (Ia), which show that there is no associated lipophilic metabolite which could penetrate the blood brain barrier and therefore affect the quantification of the density of the D1 receptor in in vivo PET imaging studies.

In yet another embodiment, the present invention relates to the use of compounds of formula (I) for quantifying *in vitro* or *ex vivo* the target engagement of D1 orthosteric ligands.

The present invention further relates to an *in vitro* or *ex vivo* method of measuring the target engagement of endogeneous or exogeneous orthosteric ligands of D1 receptor.

In yet another embodiment, the present invention relates to compounds of formula (I) as described herein for use in a method of *in vivo* imaging and measuring the target engagement of orthosteric agonists of D1-like receptor. In a particular aspect of this embodiment, the present invention relates to a compound of formula (la or Ib) for use in a method of *in vivo* imaging and measuring the target engagement of an orthosteric agonist of D1-like receptor. The term "target engagement" as used herein means an endogeneous or exogenous molecule interacting with its intended protein target (D1-like receptor) either orthosterically or allosterically and eliciting a downstream biological event.

In addition, we have surprisingly discovered that some radiolabeled compounds of formula (I) as herein described are suitable for quantifying the effect of compounds that are positive allosteric modulators of the D₁ receptor in *in vitro*, *ex vivo* and *in vivo.*

The term 'allosteric modulators' means a compound which bind to target sites distinct from the orthosteric natural agonist but which induce a conformational change in the GPCR thereby allosterically modulating the receptor function.

Positive allosteric modulators (PAM) potentiate the activity of the endogenous ligand or the activity of an exogenous orthosteric agonist.

Because D1 PAMs act on D1 receptor from a different binding site than the endogenous/exogenous ligand orthosteric site, it is desirable to have a way to quantify the actual effect of these compounds on the D1 receptor in order, for example, to be able to optimize the effective dose of the D1 PAM compound which will be needed to obtain the desired therapeutic effect on the D1-mediated diseases.

Therefore in a particular embodiment, the present invention relates to the use of compounds of formula (I) as described herein for *in vitro* or *ex vivo* quantification of the effect of D1 PAM on the D1 receptor.

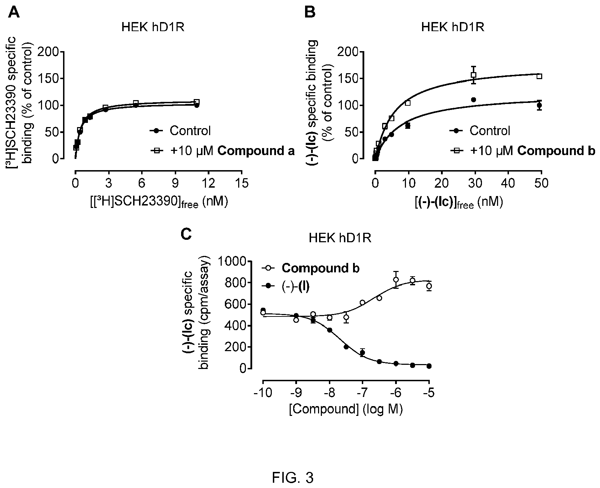

In one aspect of this embodiment, the present invention relates to the use of a compound of formula (Ic) in the *in vitro* quantitification of the effect of D1 PAM on the D1 receptor. As displayed in Figure 3, D1 PAM compound significantly improved binding properties of compound of formula (Ic).

In one aspect of this embodiment, the present invention relates to the use of a compound of formula (Ic) in the *ex vivo* quantitification of the effect of D1 PAM on the D1 receptor. In particular, the present invention relates to the use of a compound of formula (Ic) in the autoradiography quantification of the effect of D1 PAM on the D1 receptor.

Figure 4 shows by autoradiography that in the presence of a D1 PAM compound, the specific binding of compound (―)-**Ic** measured in NHP brain slices is significantly increased.

The present invention therefore also encompasses within its scope a method for *in vitro* or autoradiography detection and quantification of the D1-like receptors expression and of the effect of a D1 Allosteric Modulator in cells line expressing D1 receptor or in brain tissues.

In vitro radioligand binding or autoradiography methods comprises treating the biological sample with a radiolabeled compound according to the present invention in the presence or the absence of a D1-like or D1 orthosteric or allosteric ligand and quantifying D1-like receptor expression or allosteric modulators effect by measuring the resulting bound radiolabeled compound of the present invention.

In another particular embodiment, the present invention relates to a compound of formula (Ia) or (Ib) for use in the *in vivo* quantification of the effect of D1 PAM on the D1 receptor.

Therefore, the present invention also encompasses within its scope compounds of formula (Ia) or (Ib) for use in the *in vivo* detection and quantification of the D1-like receptors expression and of the effect of a D1 Allosteric Modulator.

In a particular embodiment, the present invention relates to a compound of formula (Ib) for use in the *in vivo* detection and quantification of the D1-like receptors expression and of the effect of a D1 Allosteric Modulator.

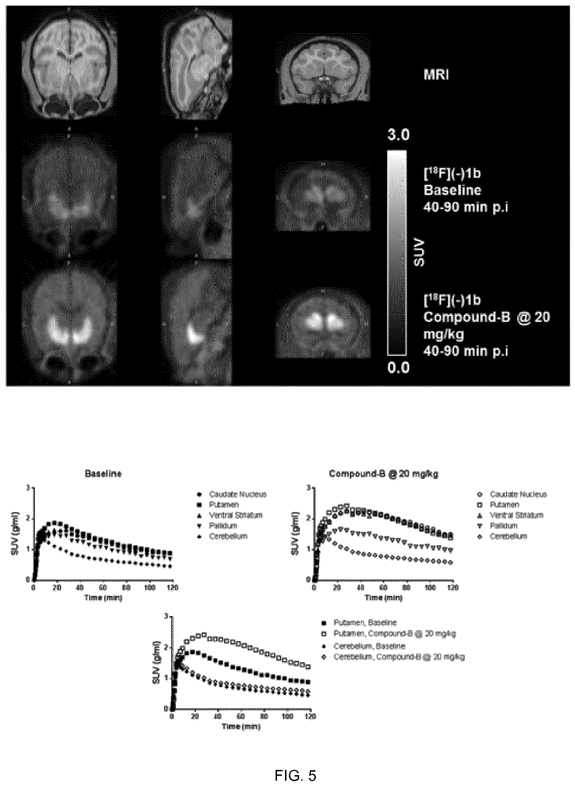

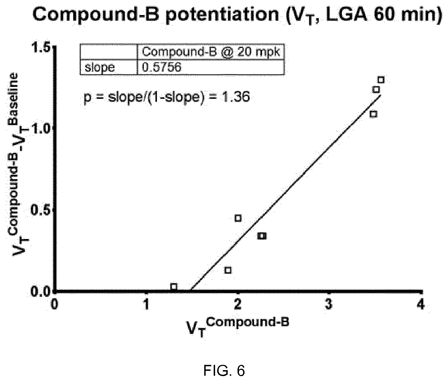

As displayed in Figure 5, D1 PAM compound significantly improved binding properties of compound of formula (lb), in particular compound of formula (―)-(Ib). The potentiation effect of such D1 PAM is shown in Figure 6.

When reference is made herein to "subject", it is intended to refer to a human, rat, mouse, cat, dog, horse, sheep, cow, human or no-human primates, avian or amphibian. Preferably reference is made to a mouse, human or non-human primate, or human.

For imaging studies, and in particular in vivo PET imaging studies, radiolabeled compounds of formula (I) or their pharmaceutically acceptable salt thereof may be administered in the form of a pharmaceutical composition.

Therefore, another embodiment of the present invention concerns a pharmaceutical composition comprising a detectable amount of a compound of formula (I), or a pharmaceutically acceptable salt thereof, in combination with a pharmaceutically acceptable diluent or carrier.

The term "detectable amount" as used herein means an amount of compound necessary to be detected by the detection method chosen. Generally such detectable quantity will be determined by the person skilled in the art based on the compound and the detection method used.

The present invention therefore also encompasses within its scope, compounds of formula (I) and their pharmaceutical composition, for use in the *in vivo* diagnostic of D1 mediated disorders.

Radiolabeled compounds of formula (I) as described herein were made according to the methods which are further detailed in the experimental section.

Compounds of formula (Ia) and (Ib) were prepared through a multi-step synthesis from compound 2-(3,5-dimethyl-2,6-dioxo-1-((2-(trimethylsilyl)ethoxy)methyl)-1 ,2,3,6-tetrahydropyrimidin-4-yl)-5-(furo[3,2-c]pyridin-4-yloxy)benzyl acetate, 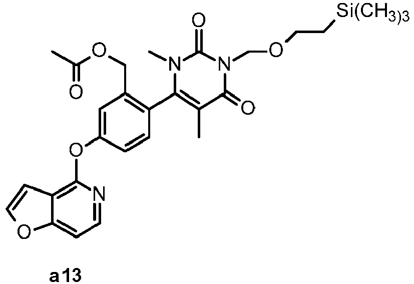 referred hereinafter as intermediate a13. Reaction conditions are further detailed in the experimental section.

Compound of formula (Ic) is prepared by tritiating compound 6-[2-(fluoromethyl)-4-(furo[3,2-c]pyridin-4-yloxy)phenyl]-1,5-dimethylpyrimidine-2,4(1H,3H)-dione (I) in the presence of Kerr's catalyst according to methods known to the person skilled in the art and as further detailed hereafter in the experimental section.

### EXPERIMENTAL SECTION

### I. Synthesis of compound of formula (I)

### a. Abbreviations/recurrent reagents

Ac: acetyl
Boc: *tert*-Butyloxycarbonyl
Brine: Saturated aqueous sodium chloride solution
Bz: benzoyl
cAMP: cyclic adenosine monophosphate
DAST: Diethylaminosulfur trifluoride
DCM: Dichloromethane
DEA: Diethylamine
DMAP : 4-dimethylaminopyridine
DMSO: Dimethylsulfoxide
dppf: 1,1 '-Bis(diphenylphosphino)ferrocene
pEC₅₀: concentration which produces 50% of the maximum response
Erel: relative efficacy
ES⁺: Electrospray Positive lonisation
ESI: Electrospray lonisation
Et: Ethyl
EtOH : Ethanol
Et₂O: Diethyl ether
EtOAc: Ethyl acetate
h: Hour
(h)D1R: human dopamine D1 receptor
(h)D5R: (human) dopamine D5 receptor
HEK: human embryonic kidney cells
HEPES: 4-(2-hydroxyethyl)-1-piperazineethanesulfonic acid
HPLC: High Pressure Liquid Chromatography
HTRF: homogenous time-resolved fluorescence
LC: Liquid Chromatography
LCMS: Liquid Chromatography Mass Spectrometry
LDA: Lithium diisopropylamide
MeOH: Methanol
min.: minutes
NHP: non-human primate
NMR: Nuclear magnetic resonance
PAM: Positive Allosteric Modulator
PrOH: isopropanol
rt: room temperature
RV: Reaction vessel
SEM: 2-(Trimethylsilyl)ethoxymethyl
SFC: Supercritical Fluid Chromatography
SPE: Solid Phase Extraction
SUV: standardized Uptake Value
TAC : Time-Activity Curve
TEA: Triethylamine
TFA: Trifluoroacetic acid
THF: Tetrahydrofuran
TLC: Thin Layer Chromatography
WFI: Water For Injection

### b. Analytical methods

All reactions involving air or moisture-sensitive reagents were performed under a nitrogen or argon atmosphere using dried solvents and glassware. Experiments requiring microwave irradiation are performed on a Biotage Initiator Sixty microwave oven upgraded with version 2.0 of the operating software. Experiments are run to reach the required temperature as quickly as possible (maximum irradiation power: 400 W, no external cooling). Commercial solvents and reagents were generally used without further purification, including anhydrous solvents when appropriate (generally Sure-Seal™ products from Aldrich Chemical Company or AcroSeal™ from ACROS Organics). In general, reactions were monitored by thin layer chromatography, HPLC or mass spectrometry analyses.

### i) HPLC analyses are performed as follows:

### Method A: Acidic

HPLC analysis is performed with Shimadzu HPLC system equipped with LC-2010 CHT module, SPD-M20A photodiode array detector (210-400 nm), by using column YMC Triart C-18 (150 × 4.6)mm 3µ. Gradient elution is done with 5 mM ammonium formate in water +0.1% formic acid (Phase A), and Acetonitrile+5%solvent A+0.1% formic acid (Phase B), with gradient 5-95% B in 8.0 min hold till 13.0 min, 5%B at 15.0 min hold till 18.0 min. HPLC flow rate: 1.0 mL/min, injection volume: 10 µL.

### Method B: Basic

HPLC analysis is performed with Shimadzu HPLC system equipped with LC-2010 CHT module, SPD-M20A photodiode array detector (210-400 nm), by using column YMC Triart C-18 (150 × 4.6)mm 3µ. Gradient elution is done with 5 mM ammonium formate in water +0.1% Ammonia (Phase A), and Acetonitrile+5%solvent A+0.1% Ammonia (Phase B), with gradient 5-95% in 8.0 min hold till 13.0 min, 5%B at 15.0 min hold till 18.0 min. HPLC flow rate.

It will be apparent to the one skilled in the art that different retention times (RT) may be obtained for LC data if different analytical conditions are used.

### ii) Mass spectrometric measurements in LCMS mode are performed as follows:

### Method A: Acidic

Shimadzu 2010EV single quadrupole mass spectrometer is used for LC-MS analysis. This spectrometer is equipped with an ESI source and LC-20AD binary gradient pump, SPD-M20A photodiode array detector (210-400 nm). Data is acquired in a full MS scan from m/z 70 to 1200 in positive and negative mode. The reverse phase analysis is carried out by using Waters XBridge C 18 (30 × 2.1)mm 2.5 µ column. Gradient elution is done with 5 mM ammonium formate in water +0.1% formic acid (Phase A) and Acetonitrile +5% solvent A +0.1% formic acid (Phase B), with gradient 5-95%B in 4.0 min hold till 5.0 min, 5% B at 5.1 min hold till 6.5 min. HPLC flow rate: 1.0 mL/min, injection volume: 5 µL.

MS parameters: Detector voltage 1.5 kV. Source block temperature 200°C. Desolvation temperature 240°C. nebulizing gas flow 1.2 L/min (Nitrogen). Data is acquired in a full MS scan from m/z 70 to 1200 in positive and negative mode.

### Method B : Basic

Shimadzu 2010EV single quadrupole mass spectrometer is used for LC-MS analysis. This spectrometer is equipped with an ESI source and LC-20AD binary gradient pump, SPD-M20A photodiode array detector (210-400 nm). Data is acquired in a full MS scan from m/z 70 to 1200 in positive and negative mode. The reverse phase analysis is carried out by using Waters XBridge C 18 (30 × 2.1)mm 2.5 µ column Gradient elution is done with 5 mM ammonium formate in water +0.1% Ammonia (solvent A),or Acetonitrile +5% solvent A+0.1% Ammonia (solvent B), with gradient 5-95% B in 4.0 min hold till 5.0 min, 5%B at 5.1 min hold till 6.5 min. HPLC flow rate: 1.0 mL/min, injection volume: 5 µL.

MS parameters: detector voltage 1.5 kV. Source block temperature 200°C. Desolvation temperature 240°C. Nebulising gas flow 1.2 L/min (Nitrogen). Data is acquired in a full MS scan from m/z 70 to 1200 in positive and negative mode.

NMR spectra are recorded on a Varian MR 400 MHz NMR Spectrometer fitted with a Linux 3.2 software with operating system Redhat enterprise Linux 5.1. and 5 mm inverse ¹H/¹³C probe head, or Varian VNMR 400 MHz NMR fitted with Linux 3.2 software with operating system Redhat enterprise Linux 6.3 and 5 mm inverse ¹H/¹³C/¹⁹F triple probe head. The compounds are studied in deuterated solvents such as DMSO-*d₆*, CDCl₃, MeOD or D₂O at a probe temperature of 300 K and at a concentration around 4-5 mg/mL. The instrument is locked on the deuterium signal of the deuterated solvent used. Chemical shifts are given in ppm downfield from TMS (tetramethylsilane) taken as internal standard.

Optical rotations ([α]_{D}) were measured on a PERKIN-ELMER polarimeter 341 in a cuvette (/=1 dm) at a 10 mg/mL concentration, at a temperature mentioned in the specific examples, at 589 nm (sodium lamp).

### iii) Preparative purification is performed by using following systems in acidic basic and neutral condition:

### System A.Waters Prep HPLC system:

Waters preparative HPLC equipped with binary pump 2545 module with 2998 PDA detector and comprising of 2767 sample manager. Waters 3100 single quadruple detector is used for detection and collection trigger.

_Shimadzu prep HPLC consists of binary LC8A pump and SPD M20A PDA detector with manual injection and manual fraction collection.

Purification is carried out by using following columns for above two systems:
Phenomenex, Synergy Fusion C18, (100 × 30)mm , 4 µ
YMC ODS (500 × 30) mm 10 µ.
YMC Triart (250 × 30)mm 10 µ.
System B. Purification on SFC

Thar SFC 100 preparative system comprised of 2545 co-solvent pump and Co2 pump, Column oven, 2767 autosampler and fraction collector, ABPR to maintain the pressure of system, 2998 PDA detector. System is controlled by Masslynx V4.1 software. Columns for SFC are selected among the ones listed below:
Virdis, 2-Ethyl pyridine (250 × 30) mm, 5µ
Virdis, CSH Fluro Phenyl ( 250 × 30) mm, 5µ
Phenomenex Luna Hilic (250 × 30) mm, 5µ
YMC, Cyano (250 × 19) mm, 5µ
YMC, Diol (250 × 30) mm, 10µ
Chiralpak IA (250 × 30) mm, 5µ

### C. Synthesis of intermediates

### C.1. Synthesis of 6-bromo-1,5-dimethyl-3-(2-trimethylsilylethoxymethyl)pyrimidine-2,4-dione a1

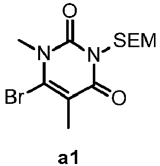

**a1** is disclosed in and prepared according to the method described in international patent application published under n°WO2014/072881.
¹H NMR (400 MHz, DMSO-*d₆*) δ 5.21 (s, 2H), 3.56 (t, *J* = 7.83 Hz, 2H), 3.52 (s, 3H), 1.99 (s, 3H), 0.83 (t, J= 7.83 Hz, 2H), -0.04 (s, 9H).

### C.2. Synthesis of 6-iodo-5-methylpyrimidine-2,4(1H,3H)-dione a6.

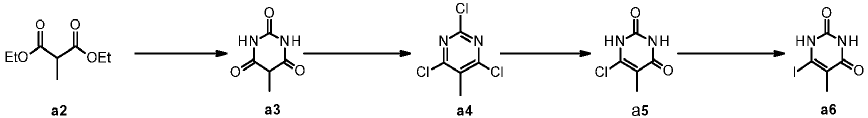

### C.2.1. Synthesis of 5-methylpyrimidine-2,4,6(1H,3H,5H)-trione a3

To a solution of Na (3.18 g, 137 mmol) in MeOH (500 mL), urea (8.28 g, 137 mmol) was added followed by addition of diethyl 2-methylpropanedioate a2 (20.0 g, 114 mmol). The reaction mixture was heated to reflux for 16h. Progress of the reaction was monitored by TLC and LCMS. After completion, the reaction mixture was concentrated under vacuum. The residue was acidified to pH 3 with a 2N aqueous solution of HCI, then cooled at -10 °C and stirred at same temperature for 3 h. The reaction mixture was filtered, washed with cold water (50 mL), Et₂O (100 mL) and hexanes (100 mL). The crude obtained was dried under vacuum to afford 15 g of 5-methylpyrimidine-2,4,6(1H,3H,5H)-trione **a3** as a white solid.
Yield (crude): 92%
LCMS (ES⁺): 143 (M+H)⁺.
¹H NMR (400 MHz, DMSO-*d₆*) δ 11.07 (s, 2H), 3.63 (q, *J* = 6.80 Hz, 1H), 1.29 (d, *J* = 6.80 Hz, 3H).

### C.2.2. Synthesis of 2,4,6-trichloro-5-methylpyrimidine a4

To a solution of 5-methylpyrimidine-2,4,6(1H,3H,5H)-trione **a3** (15.0 g, 105 mmol) in POCl₃ (158 g, 1035 mmol) was added H₃PO₄ (2 mL) and the reaction mixture was heated at 100 °C for 45 min. Progress of the reaction was monitored by TLC. After completion, the reaction mixture was poured into ice, filtered and dried under vacuum to afford 8 g of crude 2,4,6-trichloro-5-methylpyrimidine **a4** as a white solid, which was used in next step without any further purification.

### C.2.3. Synthesis of 6-chloro-5-methylpyrimidine-2,4(1H,3H)-dione a5

A stirred solution of crude 2,4,6-trichloro-5-methylpyrimidine **a4** (8.00 g, 40.8 mmol) in a 10% aqueous solution of NaOH (75 mL) was heated to reflux for 1.5 h. Progress of the reaction was monitored by TLC and LCMS. After completion, the reaction mixture was acidified to pH 2 with a 2 N aqueous solution of HCI, filtered and dried under vacuum to afford 4.8 g of 6-chloro-5-methylpyrimidine-2,4(1H,3H)-dione **a5** as a white solid, which was used in next step without any further purification.
Yield (crude): 21%
¹H NMR (400 MHz, DMSO-*d₆*) δ 11.78 (s, 1H), 11.29 (s, 1H), 1.80 (s, 3H).

### C.2.4. Synthesis of 6-iodo-5-methylpyrimidine-2,4(1H,3H)-dione a6

A stirred solution of 6-chloro-5-methylpyrimidine-2,4(1H,3H)-dione **a5** (4.80 g, 0.30 mmol) and Nal (23.4 g, 150 mmol) in HI (60 mL) was stirred in sealed tube at room temperature for 66 h. Progress of the reaction was monitored by TLC and LCMS. After completion, the reaction mixture was filtered and washed with cold water (50 mL) and dried under vacuum to afford 2.1 g of 6-iodo-5-methylpyrimidine-2,4(1H,3H)-dione **a6** was isolated as a brown solid, which was used in next step without any further purification.
LCMS (ES⁺): 253 (M+H)⁺.
¹H NMR (400 MHz, DMSO-*d₆*) δ 11.21 (s, 1H), 1.90 (s, 3H).

### C.3. Synthesis of 5-(furo[3,2-c]pyridin-4-yloxy)-2-(4,4,5,5-tetramethyl-1,3,2-dioxaborolan-2-yl)benzyl acetate a12

**a12** was prepared from compound **a7** via a multiple step synthesis according to procedures analogous to the ones described in international patent application WO2014/072881, and as specifically set out herebelow.

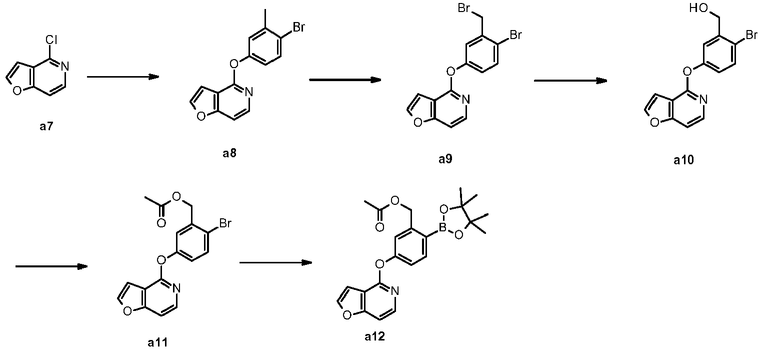

### C.3.1.Synthesis of 4-(4-bromo-3-methylphenoxy)furo[3,2-c]pyridine a8

To a solution of 4-chlorofuro[3,2-c]pyridine **a7** (12 g, 78.1 mmol) and 4-bromo-3-methylphenol (17.5 g, 93.7 mmol) in DMSO (300 mL), Cs₂CO₃ (63.4 g, 195 mmol) was added and the reaction mixture was heated at 125 °C for 16 h. Progress of the reaction was monitored by TLC and LCMS. After completion, the reaction mixture was poured onto ice and filtered. The residue was dissolved in EtOAc (100 mL) and washed with brine (2 × 100 mL). The organic layer was separated, dried over anhydrous Na₂SO₄ and concentrated under vacuum. The crude obtained was purified by triturattion with 1% Et₂O in pentane (100 mL) to afford 12.6 g of 4-(4-bromo-3-methylphenoxy)furo[3,2-c]pyridine **a8** as a beige solid.
Yield: 53%
LCMS (ES⁺): 304 (M+H)⁺.
¹H NMR (400 MHz, DMSO-*d₆*) δ 8.14 (s, 1H), 7.96 (d, J= 6.00 Hz, 1H), 7.61 (d, J= 8.80 Hz, 1H), 7.46 (d, *J* = 5.60 Hz, 1H), 7.25 (d, J= 1.20 Hz, 1H), 7.10 (s, 1H), 7.04-6.98 (m, 1H), 2.34 (s, 3H).

### C.3.2. Synthesis of 4-(4-bromo-3-(bromomethyl)phenoxy)furo[3,2-c]pyridine a9

To a solution of 4-(4-bromo-3-methylphenoxy)furo[3,2-c]pyridine **a8** (12.5 g, 41.1 mmol) in CCl₄ (250 mL) were added NBS (8.05 g, 45.2 mmol) and Bz₂O₂ (1.99 g, 8.22 mmol). The reaction mixture was heated at 80 °C for 12 h. Progress of the reaction was monitored by TLC and LCMS. After completion, the reaction mixture was diluted with DCM (100 mL) and water (100 mL). The organic layer was washed with a 1N aqueous solution of NaOH (50 mL) and brine (50 mL), dried over anhydrous Na₂SO₄ and concentrated under vacuum to afford 22 g of 4-(4-bromo-3-(bromomethyl)phenoxy)furo[3,2-c]pyridine **a9** as a brown semi solid, which was used in next step without any further purification.
LCMS (ES⁺): 284 (M+H)⁺.

### C.3.3. Synthesis of (2-bromo-5-(furo[3,2-c]pyridin-4-yloxy)phenyl)methanol a10

To a solution of 4-(4-bromo-3-(bromomethyl)phenoxy)furo[3,2-c]pyridine **a9** (22.0 g, 57.4 mmol) in DMF (80 mL), NaOAc (23.0 g, 287 mmol) was added and the reaction mixture was heated at 80 °C for 3 h. Progress of the reaction was monitored by TLC and LCMS. After completion, the reaction mixture was diluted with DCM (100 mL) and water (50 mL). The organic layer was dried over anhydrous Na₂SO₄ and concentrated under vacum. The residue was dissolved in MeOH (50 mL) followed by addition of a 1N aqueous solution of NaOH (20 mL). The reaction mixture was stirred at room temperature for 1 h. The reaction mixture was concentrated under vacuum. The residue was diluted with water (100 mL) and extracted with EtOAc (3 × 40 mL). The organic layer was dried over anhydrous Na₂SO₄ and concentrated under vacuum. The residue was purified by column chromatography using from 20% EtOAc in hexanes as eluent to yield 3.5 g of (2-bromo-5-(furo[3,2-c]pyridin-4-yloxy)phenyl)methanol **a10** as a white solid.
Yield: 19%
LCMS (ES⁺): 320 (M+H)⁺.
¹H NMR (400 MHz, DMSO-*d₆*) δ 8.14 (d, *J* = 2.40 Hz, 1H), 7.96 (d, *J* = 5.60 Hz, 1H), 7.61 (d, *J* = 8.80 Hz, 1H), 7.48 (d, *J* = 6.00 Hz, 1H), 7.32 (d, *J* = 2.80 Hz, 1H), 7.12-7.08 (m, 2H), 5.51 (t, *J* = 5.60 Hz, 1H), 4.51 (d, *J* = 5.60 Hz, 2H).

### C.3.4. Synthesis of 2-bromo-5-(furo[3,2-c]pyridin-4-yloxy)benzyl acetate a11

To a solution of (2-bromo-5-(furo[3,2-c]pyridin-4-yloxy)phenyl)methanol **a10** (3.50 g, 11.0 mmol) in THF (50 mL), pyridine (2.60 g, 33.0 mmol) was added at 0 °C and the reaction mixture was stirred at same temperature for 10 min. Acetylchloride (1.73 g, 22.0 mmol) was added dropwise at 0 °C and the reaction mixture was heated at 100 °C for 16 h. Progress of the reaction was monitored by TLC and LCMS. After completion, the reaction mixture was neutralised with an aqueous saturated solution of NaHCO₃ and extracted with EtOAc (50 mL). The organic layer was successively washed with water (50 mL) and brine (10 mL), dried over anhydrous Na₂SO₄ and concentrated under vacuum. The residue was purified by column chromatography using from 2 to 5% EtOAc in hexanes as eluent to afford 4 g of 2-bromo-5-(furo[3,2-c]pyridin-4-yloxy)benzyl acetate **a11** as colourless liquid.
Yield: 98%
LCMS (ES⁺): 362 (M+H)⁺.
¹H NMR (400 MHz, DMSO-*d₆*) δ 8.14 (d, *J* = 2.00 Hz, 1H), 7.95 (d, *J* = 6.00 Hz, 1H), 7.69 (d, *J* = 8.80 Hz, 1H), 7.49-7.45 (m, 1H), 7.35 (d, *J* = 2.80 Hz, 1H), 7.20-7.17 (m, 1H), 7.14-7.11 (m, 1H), 5.10 (s, 2H), 2.07 (s, 3H).

### C.3.5. Synthesis of 5-(furo[3,2-c]pyridin-4-yloxy)-2-(4,4,5,5-tetramethyl-1,3,2-dioxaborolan-2-yl)benzyl acetate a12

To a solution of 2-bromo-5-(furo[3,2-c]pyridin-4-yloxy)benzyl acetate **a11** (4 g, 11.1 mmol) and 4,4,5,5-tetramethyl-2-(4,4,5,5-tetramethyl-1,3,2-dioxaborolan-2-yl)-1,3,2-dioxaborolane (3.66 g, 14.4 mmol) in dioxane (50 mL) was added KOAc (3.26 g, 33.3 mmol). The reaction mixture was purged with argon for 30 min, then PdCl₂(dppf).DCM (2.70 g, 3.33 mmol) was added and the reaction mixture was purged again with argon for 5 min. The reaction mixture was heated at 110 °C for 16 h. Progress of the reaction was monitored by TLC and LCMS. After completion, the reaction mixture was diluted with EtOAc (50 mL) and filtered through Celite^{®}. The filtrate was concentrated under vacuum. The residue was purified by column chromatography using from 10 to 12% EtOAc in hexanes to afford 4.1 g of 5-(furo[3,2-c]pyridin-4-yloxy)-2-(4,4,5,5-tetramethyl-1,3,2-dioxaborolan-2-yl)benzyl acetate **a12** was isolated as an off-white solid.
LCMS (ES⁺): 410 (M+H)⁺.

### C.4. Synthesis of 2-(3,5-dimethyl-2,6-dioxo-1-((2-(trimethylsilyl)ethoxy)methyl)-1,2,3,6-tetrahydropyrimidin-4-yl)-5-(furo[3,2-c]pyridin-4-yloxy)benzyl acetate a13, a13_Atropoisomer_1 and a13_Atropoisomer_2

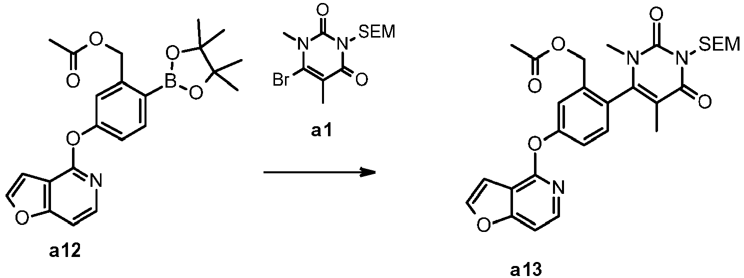

To a solution of 5-(furo[3,2-c]pyridin-4-yloxy)-2-(4,4,5,5-tetramethyl-1,3,2-dioxaborolan-2-yl)benzyl acetate **a12** (200 mg, 0.49 mmol) and 6-bromo-1,5-dimethyl-3-(2-trimethylsilylethoxymethyl)pyrimidine-2,4-dione **a1** (130 mg, 0.38 mmol) in dioxane (9 mL) and water (1 mL) was added K₂CO₃ (160 mg, 1.13 mmol). The reaction mixture was purged with argon for 30 min. PdCl₂(dppf).DCM (90 mg, 0.11 mmol) was added and the reaction mixture was purged again with argon for 5 min. The reaction mixture was heated under microwave irradiations at 120 °C for 2 h. Progress of the reaction was monitored by TLC and LCMS. The reaction was repeated on 13 × 0.20 g and the crude mixtures of the 14 reactions were combined. The reaction mixture was diluted with EtOAc (150 mL), filtered through Celite^{®} and washed with EtOAc (250 mL). The filtrate was concentrated under vacuum. The residue was purified by column chromatography using from 20 to 40% EtOAc in hexanes as eluent to afford 827 mg of 2-(3,5-dimethyl-2,6-dioxo-1-((2-(trimethylsilyl)ethoxy)methyl)-1,2,3,6-tetrahydropyrimidin-4-yl)-5-(furo[3,2-c]pyridin-4-yloxy)benzyl acetate **a13** as an off-white solid, which is a racemic mixture of atropisomers (+) and (-).
Yield: 31%
LCMS (ES⁺): 494 (M+H-SiMe₃)⁺.
¹H NMR (400 MHz, DMSO-*d₆*) δ 8.18 (d, *J* = 1.96 Hz, 1H), 8.05 (d, *J* = 5.87 Hz, 1H), 7.54 (dd, *J* = 5.87, 0.98 Hz, 1H), 7.49 (s, 1H), 7.44 (s, 2H), 7.14-7.12 (m, 1H), 5.76 (s, 2H), 5.32 (s, 2H), 3.68-3.59 (m, 2H), 2.97 (s, 3H), 1.95 (s, 3H), 1.55 (s, 3H), 1.17 (t, J= 7.09 Hz, 2H), -0.01 (s, 9H).

Chiral separation (LC, YMC Chiralart Cellulose-SC, 250*4.6 mm, 1 mL/min, 252 nm, 25 °C, eluent: 50% n-hexanes (with 0.1% DEA), 50% iPrOH) of 500 mg of racemate 2-(3,5-dimethyl-2,6-dioxo-1-((2-(trimethylsilyl)ethoxy)methyl)-1,2,3,6-tetrahydropyrimidin-4-yl)-5-(furo[3,2-c]pyridin-4-yloxy)benzyl acetate **a13** afforded:
- 151 mg of 2-(3,5-dimethyl-2,6-dioxo-1-((2-(trimethylsilyl)ethoxy)methyl)-1,2,3,6-tetrahydropyrimidin-4-yl)-5-(furo[3,2-c]pyridin-4-yloxy)benzyl acetate (**a13_atropisomer 1**) as a white solid.
   Yield: 30%
   LCMS (ES⁺): 494 (M+H-SiMe₃)⁺.

Chiral analysis (LC, YMC Chiralart Cellulose-SC, 250*4.6 mm, 1 mL/min, 252 nm, eluent: 50% n-hexanes (with 0.1% DEA), 50% iPrOH) RT 28.93 min, 98.5% ee.
- 152 mg of 2-(3,5-dimethyl-2,6-dioxo-1-((2-(trimethylsilyl)ethoxy)methyl)-1,2,3,6-tetrahydropyrimidin-4-yl)-5-(furo[3,2-c]pyridin-4-yloxy)benzyl acetate (**a13_atropisomer 2)** as a white solid.
   Yield: 30%
   LCMS (ES⁺): 494 (M+H-SiMe₃)⁺.

Chiral analysis (LC, YMC Chiralart Cellulose-SC, 250*4.6 mm, 1 mL/min, 252 nm, eluent: 50% n-hexanes (with 0.1% DEA), 50% iPrOH) RT 31.28 min, 92.5% ee.

Particular configuration (+) or (-) of the respective atropoisomers was not assigned at this stage.

### D. Synthesis of compounds of formula (I)

### D.1. Synthesis of 6-(2-(fluoromethyl)-4-(furo[3,2-c]pyridin-4-yloxy)phenyl)-1,5-dimethylpyrimidine-2,4(1H,3H)-dione (+)-(I) and (-)-(I).

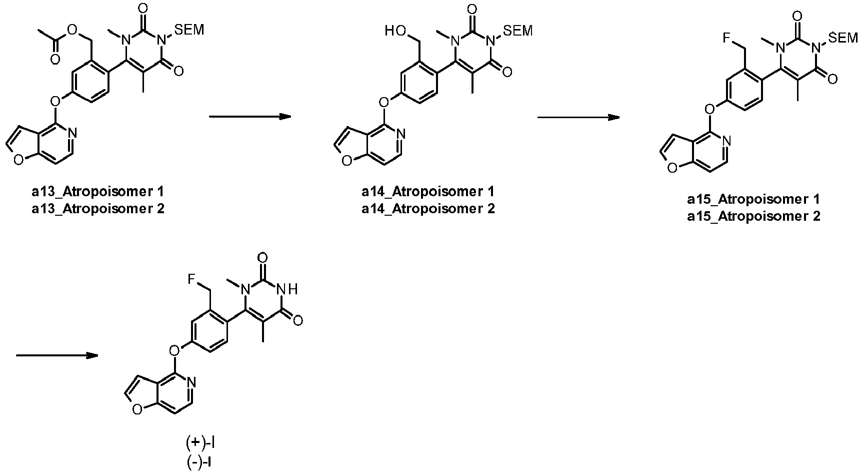

### D.1.1. Synthesis of 6-(4-(furo[3,2-c]pyridin-4-yloxy)-2-(hydroxymethyl)phenyl)-1,5-dimethyl-3-((2-(trimethylsilyl)ethoxy)methyl)pyrimidine-2,4(1H,3H)-dione a14_Atropoisomer 1 and of a14_Atropoisomer 2

To a solution of 2-(3,5-dimethyl-2,6-dioxo-1-((2-(trimethylsilyl)ethoxy)methyl)-1,2,3,6-tetrahydropyrimidin-4-yl)-5-(furo[3,2-c]pyridin-4-yloxy)benzyl acetate **a13_Atropoisomer 1** (151 mg, 0.27 mmol) in THF (7.71 mL) and water (860 µL) was added NaOH (50 mg, 1.36 mmol). The reaction mixture was stirred at room temperature for 16 h. Progress of the reaction was monitored by TLC and LCMS. After completion, the reaction mixture was concentrated under vacuum. The residue was diluted with water (15 mL) and extracted with EtOAc (2 × 50 mL). The organic layer was washed with brine (10 mL), dried over anhydrous Na₂SO₄ and concentrated under vacuum to afford 124 mg of 6-(4-(furo[3,2-c]pyridin-4-yloxy)-2-(hydroxymethyl)phenyl)-1,5-dimethyl-3-((2-(trimethylsilyl)ethoxy)methyl)pyrimidine-2,4(1H,3H)-dione **a14_Atropoisomer 1** as a light brown liquid, which was used in next step without any further purification.
Yield (crude): 90%
LCMS (ES⁺): 510 (M+H)⁺.

### 6-(4-(furo[3,2-c]pyridin-4-yloxy)-2-(hydroxymethyl)phenyl)-1,5-dimethyl-3-((2-(trimethylsilyl)ethoxy)methyl)pyrimidine-2,4(1H,3H)-dione a14 Atropoisomer 2

Compound **a14_Atropoisomer 2**_may be synthetized according to the same method using 2-(3,5-dimethyl-2,6-dioxo-1-((2-(trimethylsilyl)ethoxy)methyl)-1,2,3,6-tetrahydropyrimidin-4-yl)-5-(furo[3,2-c]pyridin-4-yloxy)benzyl acetate **a13_Atropoisomer 1** as starting material.
Yield(crude): 93%.
LCMS (ES⁺): 510 (M+H)⁺.

Particular configuration (+) or (-) of the respective atropoisomers was not assigned at this stage.

### D.1.2. Synthesis of 6-(2-(fluoromethyl)-4-(furo[3,2-c]pyridin-4-yloxy)phenyl)-1,5-dimethyl-3-((2-(trimethylsilyl)ethoxy)methyl)pyrimidine-2,4(1H,3H)-dione a15_Atropoisomer 1 and a15_Atropoisomer 2

To a solution of 6-(4-(furo[3,2-c]pyridin-4-yloxy)-2-(hydroxymethyl)phenyl)-1,5-dimethyl-3-((2-(trimethylsilyl)ethoxy)methyl)pyrimidine-2,4(1H,3H)-dione **a14_Atropoisomer 1** (0.13 g, 0.25 mmol) in DCM (3 mL) was added DAST (0.20 g, 1.23 mmol) at 0 °C. The reaction mixture was stirred at room temperature for 2 h. Progress of the reaction was monitored by TLC and LCMS. After completion, the reaction mixture was quenched with an aqueous saturated solution of NaHCO₃ (2 mL) and extracted with EtOAc (2 × 10 mL). The organic layer was successively washed with water (10 mL) and brine (5 mL), dried over anhydrous Na₂SO₄ and concentrated under vacuum. The residue was purified by column chromatography using from 20 to 30% EtOAc in hexanes as eluent to afford 74 mg of 6-(2-(fluoromethyl)-4-(furo[3,2-c]pyridin-4-yloxy)phenyl)-1,5-dimethyl-3-((2-(trimethylsilyl)ethoxy)methyl)pyrimidine-2,4(1H,3H)-dione **a15_Atropoisomer 1** as an off-white solid.
Yield: 59%
LCMS (ES⁺): 512 (M+H)⁺.
¹H NMR (400 MHz, DMSO-*d₆*) δ 8.18 (d, *J* = 2.45 Hz, 1H), 8.05 (d, *J* = 5.87 Hz, 1H), 7.58-7.53 (m, 2H), 7.48 (s, 2H), 7.16-7.12 (m, 1H), 5.39 (d, *J* = 2.45 Hz, 1H), 5.32 (s, 2H), 5.27 (s, 1H), 3.69-3.62 (m, 2H), 2.93 (s, 3H), 1.54 (s, 3H), 0.92-0.84 (m, 2H), 0.03 (s, 9H).

### 6-(2-(fluoromethyl)-4-(furo[3,2-c]pyridin-4-yloxy)phenyl)-1,5-dimethyl-3-((2-(trimethylsilyl)ethoxy)methyl)pyrimidine-2,4(1H,3H)-dione a15 Atropoisomer 2

Compound **a15_Atropoisomer 2** may be synthetized according to the same method using 6-(4-(furo[3,2-c]pyridin-4-yloxy)-2-(hydroxymethyl)phenyl)-1,5-dimethyl-3-((2-(trimethylsilyl)ethoxy)methyl)pyrimidine-2,4(1H,3H)-dione **a14_Atropoisomer 2** as starting material.
Yield: 58%
LCMS (ES⁺): 512 (M+H)⁺.
¹H NMR (400 MHz, DMSO-*d₆*) δ 8.18 (d, *J* = 2.45 Hz, 1H), 8.05 (d, *J* = 5.87 Hz, 1H), 7.58-7.53 (m, 2H), 7.48 (s, 2H), 7.16-7.12 (m, 1H), 5.39 (d, *J* = 2.45 Hz, 1H), 5.32 (s, 2H), 5.27 (s, 1H), 3.69-3.62 (m, 2H), 2.93 (s, 3H), 1.54 (s, 3H), 0.92-0.84 (m, 2H), 0.03 (s, 9H).

Particular configuration (+) or (-) of the respective atropoisomers was not assigned at this stage.

### D.1.3. Synthesis of 6-(2-(fluoromethyl)-4-(furo[3,2-c]pyridin-4-yloxy)phenyl)-1,5-dimethylpyrimidine-2,4(1H,3H)-dione (+)-(I) and (-)-(I)

To a solution of 6-(2-(fluoromethyl)-4-(furo[3,2-c]pyridin-4-yloxy)phenyl)-1,5-dimethyl-3-((2-(trimethylsilyl)ethoxy)methyl)pyrimidine-2,4(1H,3H)-dione **a15_Atropoisomer 1** (0.07 g, 0.15 mmol) in DCM (1 mL) was added TFA (1 mL) at 0 °C. The reaction mixture was stirred at room temperature for 2 h. Progress of the reaction was monitored by TLC and LCMS. After completion, the reaction mixture was concentrated under vacuum. The residue was dissolved in CH₃CN (1 mL) and a 25% aqueous solution of ammonia (1 mL). The reaction mixture was stirred for 30 min, then filtered, washed with cold water (2 mL) and dried under vacuum. The residue was purified by trituration with DCM:pentane (1:4, 2.5 mL) and dried under vacuum to afford 20 mg of atropisomer (+) of 6-(2-(fluoromethyl)-4-(furo[3,2-c]pyridin-4-yloxy)phenyl)-1,5-dimethylpyrimidine-2,4(1H,3H)-dione **(+)-(I)** as an off-white solid.
Yield: 36%
HPLC Purity: 97.5%
LCMS (ES⁺): 382 (M+H)⁺.
¹H NMR (400 MHz, DMSO-*d₆*) δ 11.48 (s, 1H), 8.16-8.19 (m, 1H), 8.05 (d, *J* = 5.87 Hz, 1H), 7.56-7.52 (m, 2H), 7.49-7.44 (m, 2H), 7.13 (d, *J* = 1.47 Hz, 1H), 5.39 (d, *J* = 3.42 Hz, 1H), 5.27 (d, *J* = 2.93 Hz, 1H), 2.86 (s, 3H), 1.48 (s, 3H).
[α]_{D} (MeOH, 30°C) = +14.7

### 6-(2-(fluoromethyl)-4-(furo[3,2-c]pyridin-4-yloxy)phenyl)-1,5-dimethylpyrimidine-2,4(1H,3H)-dione (I) atropisomer (-), ((-)-I)

Compound **(-)-I** may be synthetized according to the same method using 6-(2-(fluoromethyl)-4-(furo[3,2-c]pyridin-4-yloxy)phenyl)-1,5-dimethyl-3-((2-(trimethylsilyl)ethoxy)methyl)pyrimidine-2,4(1H,3H)-dione **a15_Atropoisomer 2** as starting material.
HPLC Purity: 99.8%
LCMS (ES⁺): 382 (M+H)⁺.
¹H NMR (400 MHz, DMSO-*d₆*) δ 11.48 (s, 1H), 8.19-8.16 (m, 1H), 8.05 (d, *J* = 5.87 Hz, 1H), 7.56-7.52 (m, 2H), 7.46 (brs, 2H), 7.13 (d, *J* = 1.47 Hz, 1H), 5.39 (d, *J* = 3.42 Hz, 1H), 5.27 (d, *J* = 2.93 Hz, 1H), 2.86 (s, 3H), 1.48 (s, 3H).
[α]_{D} (MeOH, 30°C) = -8.3

### D.2. Synthesis of atropoisomer (-) of [¹¹C]-6-(2-(fluoromethyl)-4-(furo[3,2-c]pyridin-4-yloxy)phenyl)-1,5-dimethylpyrimidine-2,4(1H,3H)-dione (Ia), (-)-(Ia)

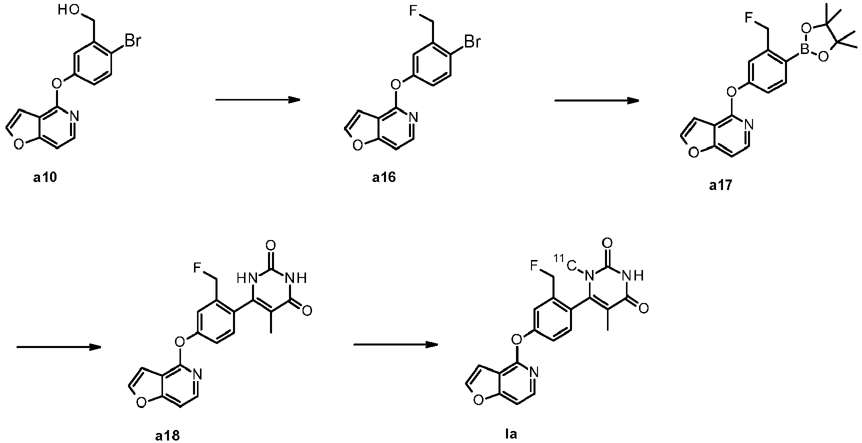

### D.2.1. Synthesis of 4-(4-bromo-3-(fluoromethyl)phenoxy)furo[3,2-c]pyridine a16

To a solution of (2-bromo-5-(furo[3,2-c]pyridin-4-yloxy)phenyl)methanol **a10** (5g, 15.7 mmol) in DCM (100 mL) was added DAST (10 g, 62.8 mmol) at 0 °C, then the reaction mixture was stirred at room temperature for 2 h. Progress of the reaction was monitored by TLC and LCMS. After completion, the reaction mixture was quenched with an aqueous saturated solution of NaHCO₃ (20 mL) and extracted with DCM (100 mL). The organic layer was dried over anhydrous Na₂SO₄ and concentrated under vacuum. The residue was purified by column chromatography using from 10 to 15% EtOAc in hexanes as eluent to afford 2.1 g of 4-(4-bromo-3-(fluoromethyl)phenoxy)furo[3,2-c]pyridine **a16** as an off-white solid.
Yield: 41%
LCMS (ES⁺): 322 (M+H)⁺.
¹H NMR (400 MHz, CDCl₃) δ 7.99 (d, *J* = 6.00 Hz, 1H), 7.68-7.64 (m, 1H), 7.59 (d, J= 8.80 Hz, 1H), 7.38-7.36 (m, 1H), 7.22 (d, *J* = 5.60 Hz, 1H), 7.14 - 7.10 (m, 1H), 6.91 (s, 1H), 5.46 (d, *J* = 46.8 Hz, 2H).

### D.2.2. Synthesis of 4-(3-(fluoromethyl)-4-(4,4,5,5-tetramethyl-1,3,2-dioxaborolan-2-yl)phenoxy)furo[3,2-c]pyridine a17

To a solution of 4-(4-bromo-3-(fluoromethyl)phenoxy)furo[3,2-c]pyridine **a16** (2 g, 6.23 mmol) and 4,4,5,5-tetramethyl-2-(4,4,5,5-tetramethyl-1,3,2-dioxaborolan-2-yl)-1,3,2-dioxaborolane (2.05 g, 8.09 mmol) in dioxane (50 mL) was added KOAc (1.83 g, 18.6 mmol), then the reaction was purged with argon for 30 min. PdCl₂(dppf) (1.52 g, 1.86 mmol) was added and the reaction mixture was purged again with argon for 5 min. The reaction mixture was heated at 100 °C for 12 h. Progress of the reaction was monitored by TLC and LCMS. After completion, the reaction mixture was filtered through Celite^{®} and washed with EtOAc (100 mL). The filtrate was concentrated under vacuum. The residue was purified by column chromatography using 10% EtOAc in hexanes as eluent to afford 2.1 g of 4-(3-(fluoromethyl)-4-(4,4,5,5-tetramethyl-1,3,2-dioxaborolan-2-yl)phenoxy)furo[3,2-c]pyridine **a17** as an off-white solid.
Yield: 91%
¹H NMR (400 MHz, DMSO-*d₆*) δ 8.16-8.14 (m, 1H), 7.98 (d, *J* = 5.20 Hz, 1H), 7.79 (d, *J* = 8.40 Hz, 1H), 7.49 (d, *J* = 6.00 Hz, 1H), 7.32-7.28 (m, 1H), 7.24-7.20 (m, 1H), 7.09 (s, 1H), 5.65 (d, *J* = 47.6 Hz, 2H), 1.31 (s, 12H).

### D.2.3. Synthesis of 6-(2-(fluoromethyl)-4-(furo[3,2-c]pyridin-4-yloxy)phenyl)-5-methylpyrimidine-2,4(1H,3H)-dione a18

To a solution of 4-(3-(fluoromethyl)-4-(4,4,5,5-tetramethyl-1,3,2-dioxaborolan-2-yl)phenoxy)furo[3,2-c]pyridine **a17** (100 mg, 0.27 mmol) and 6-iodo-5-methylpyrimidine-2,4(1H,3H)-dione **a6** (130 mg, 0.54 mmol) in dioxane (10 mL) and water (1 mL) was added K₂CO₃ (110 mg, 0.81 mmol) and the reaction was purged with argon for 30 min. PdCl₂(dppf) (60 mg, 0.08 mmol) was added and the reaction mixture was purged again with argon for 5 min. The reaction mixture was heated at 80 °C for 1 h. Progress of the reaction was monitored by TLC and LCMS. After completion, the reaction mixture was filtered through Celite^{®}. The filtrate was concentrated under vacuum. The residue was purified by column chromatography using from 50 to 60% EtOAc in hexanes as eluent to afford 20 mg of 6-(2-(fluoromethyl)-4-(furo[3,2-c]pyridin-4-yloxy)phenyl)-5-methylpyrimidine-2,4(1H,3H)-dione **a18** as an off-white solid.
Yield: 21%
HPLC Purity: 98.4%
LCMS (ES⁺): 368 (M+H)⁺.
¹H NMR (400 MHz, DMSO-*d₆*) δ 11.18 (s, 1H), 10.85 (s, 1H), 8.17 (d, *J* = 2.00 Hz, 1H), 8.02 (d, *J* = 6.00 Hz, 1H), 7.52 (d, *J* = 6.00 Hz, 1H), 7.45-7.37 (m, 3H), 7.11 (d, *J* = 1.60 Hz, 1H), 5.36 (d, *J* = 46.8 Hz, 2H), 1.51 (s, 3H).

### D.2.4. Synthesis of atropisomer (-) of [¹¹C]-6-(2-(fluoromethyl)-4-(furo[3,2-c]pyridin-4-yloxy)phenyl)-1,5-dimethylpyrimidine-2,4(1H,3H)-dione (Ia), (-)-(Ia).

[¹¹C]-(6-(2-(fluoromethyl)-4-(furo[3,2-c]pyridin-4-yloxy)phenyl)-1,5-dimethylpyrimidine-2,4(1H,3H)-dione) as a mixture of two atropoisomers was prepared starting from the **a18** precursor and [¹¹C]methane. The [¹¹C]methane was produced in a GE PETtrace cyclotron by irradiating the gas target containing a mixture of 10% hydrogen in nitrogen gas with a 16.5 MeV proton beam, producing [¹¹C]methane via ¹⁴N(p, α)¹¹C nuclear reaction and subsequent in-target reaction of carbon-11 with nitrogen to form methane.

Produced [¹¹C]methane was then trapped at ca -180 °C on a HayeSep trap that is subsequently flushed with helium to remove traces of hydrogen and nitrogen. The trapped [¹¹C]methane was then released under heating into a recirculation system where it was reacted with iodine vapor at approximately 720 °C to produce [¹¹C]methyl iodide. After recirculation, the [¹¹C]methyl iodide was passed in a stream of helium through a column containing heated silver triflate on a inert carrier to produce the labeling agent, [¹¹C]methyl triflate. This in turn, was trapped in a solution containing the unlabeled precursor **a18,** 0.2-0.4 mg, 4-5 µl of 0.5M aq. NaOH and 600-700 µl of acetone.

After ca. 120 seconds the reaction mixture was diluted with water and transferred onto a semi-preparative XBridge C18 column (200×10 mm, 5 µm) and eluted with a mixture of acetonitrile and aqueous NH₄OH (0.15%) 29:71 at a flow of 7 mL/min. The effluent was monitored with radioactivity and UV detector connected sequentially, with UV detector set to 254 nm. The peak containing radioactive labeled product was collected, diluted in ca. 50 ml of sterile water and passed though Waters Oasis 3cc SPE cartridge to trap the product. The cartridge was then washed with 8 mL of sterile water and the product eluted with ca. 1.1 ml of 96% ethanol into a vial containing 12 ml of saline. The product was then filtered though a 0.22 µm sterile filter (Millex GV). Sample for quality control was taken from the product vial after filtration. Radiochemical purity was determined using a standard HPLC system, with radioactivity and UV detector connected in series, using analytical XBridge C18 column (150x4.6 mm, 5 µm) with acetonitrile/aq. NH₄OH (0.15%) 25:75 as mobile phase, with UV detector set to 254 nm.

As the two atropoisomers cannot be separated on a regular C18 HPLC column, a chiral separation method was developed for their separation.

For the isolation of atropisomer (-) of (Ia), the semi-preparative HPLC was performed using Chiralpak IA column (250x10 mm, 5 µm) using acetonitrile/ethanol 7:93 mixture as mobile phase, at a flow of 5 mL/min, with UV detector set to 254 nm. The reaction itself and the product formulation were performed as described above.

The radiochemical and atropoisomeric purities were determined using a standard HPLC system equipped with Chiralpak IA-3 analytical clumn, eluted with MeCN/EtOH 7:93 mobile phase at a flow of 1 ml/min, with UV detector set to 254 nm (retention time of (-)-Ia = 7.2 minutes, ee>99%).

### D.3. Synthesis of atropisomer (-) of [¹⁸F]-6-(2-(fluoromethyl)-4-(furo[3,2-c]pyridin-4-yloxy)phenyl)-1,5-dimethylpyrimidine-2,4(1H,3H)-dione (Ib), (-)-(Ib)

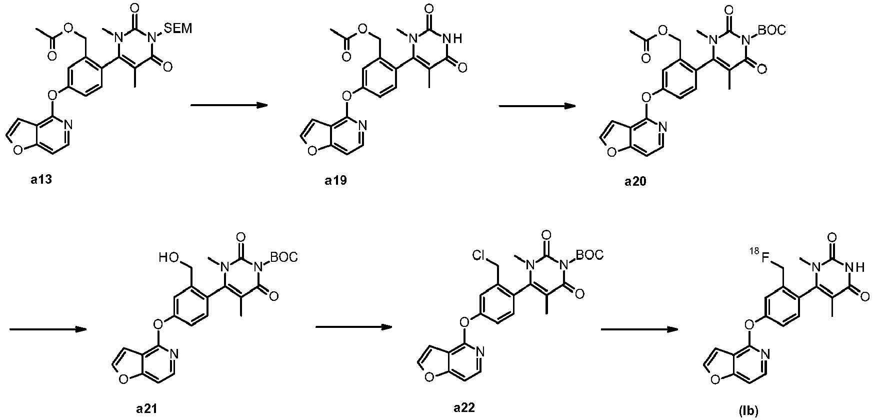

### D.3.1. Synthesis of 2-(3,5-dimethyl-2,6-dioxo-1,2,3,6-tetrahydropyrimidin-4-yl)-5-(furo[3,2-c]pyridin-4-yloxy)benzyl acetate a19

To a solution of 2-(3,5-dimethyl-2,6-dioxo-1-((2-(trimethylsilyl)ethoxy)methyl)-1,2,3,6-tetrahydropyrimidin-4-yl)-5-(furo[3,2-c]pyridin-4-yloxy)benzyl acetate **a13** (2.60 g, 4.71 mmol) in DCM (50 mL) was added TFA (26.0 mL, 350 mmol) was added at 0 °C, then the reaction mixture was stirred at room temperature for 2 h. Progress of the reaction was monitored by TLC and LCMS. After completion, the reaction mixture was concentrated under vacuum. The residue was dissolved in a mixture acetonitrile:ammonia (1:1, 10 mL) and stirred for 30 min. The reaction mixture was filtered, washed with cold water (20 mL) and pentane (20 mL) and dried under vacuum to afford 1.6 g of 2-(3,5-dimethyl-2,6-dioxo-1,2,3,6-tetrahydropyrimidin-4-yl)-5-(furo[3,2-c]pyridin-4-yloxy)benzyl acetate **a19** as an off-white solid, which was used in next step without any further purification.
Yield(crude): 81%
LCMS (ES⁺): 422 (M+H)⁺.
¹H NMR (400 MHz, DMSO-*d₆*) δ 11.47(s, 1H), 8.18 (d, J= 1.96 Hz, 1H), 8.05 (d, *J* = 5.87 Hz, 1H), 7.54 (dd, J= 5.87, 0.98 Hz, 1H), 7.48 (s, 1H), 7.45-7.41 (m, 2H), 7.13 (s, 1H), 5.02-4.97 (m, 1H), 4.97-4.91 (m, 1H), 2.90 (s, 3H), 1.97 (s, 3H), 1.50 (s, 3H).

D.3.2. Synthesis of tert-butyl 4-(2-(acetoxymethyl)-4-(furo[3,2-c]pyridin-4-yloxy)phenyl)-3,5-dimethyl-2,6-dioxo-3,6-dihydropyrimidine-1(2H)-carboxylate **a20** 2-(3,5-dimethyl-2,6-dioxo-1,2,3,6-tetrahydropyrimidin-4-yl)-5-(furo[3,2-c]pyridin-4-yloxy)benzyl acetate **a19** (1.60 g, 3.80 mmol), (Boc)₂O (1.57 mL, 6.83 mmol), triethylamine (0.64 mL, 4.56 mmol) and DMAP (0.05 g, 0.38 mmol) were dissolved in THF (33.3 mL) and the reaction mixture was heated at 70 °C for 16 h. Progress of the reaction was monitored by TLC and LCMS. After completion, the reaction mixture was concentrated under vacuum. The residue was purified by column chromatography using from 70 to 80% EtOAc in hexanes as eluent to afford 1.1 g of tert-butyl 4-(2-(acetoxymethyl)-4-(furo[3,2-c]pyridin-4-yloxy)phenyl)-3,5-dimethyl-2,6-dioxo-3,6-dihydropyrimidine-1(2H)-carboxylate **a20** as an off-white solid.
Yield: 56%
LCMS (ES⁺): 522 (M+H)⁺.
¹H NMR (400 MHz, DMSO-*d₆*) δ 8.17 (d, *J* = 1.96 Hz, 1H), 8.04 (d, *J* = 5.87 Hz, 1H), 7.52 (d, *J* = 5.87 Hz, 1H), 7.40 (d, *J* = 8.31Hz, 2H), 7.34-7.30 (m, 1H), 7.10 (d, *J* = 1.47 Hz, 1H), 4.33-4.38 (m, 2H), 2.94 (s, 3H), 1.95 (s, 3H), 1.55 (s, 9H), 1.52 (s, 3H).

### D.3.3. Synthesis of tert-butyl 4-(4-(furo[3,2-c]pyridin-4-yloxy)-2-(hydroxymethyl)phenyl)-3,5-dimethyl-2,6-dioxo-3,6-dihydropyrimidine-1(2H)-carboxylate a21

A solution of tert-butyl 4-(2-(acetoxymethyl)-4-(furo[3,2-c]pyridin-4-yloxy)phenyl)-3,5-dimethyl-2,6-dioxo-3,6-dihydropyrimidine-1(2H)-carboxylate **a20** (1.1 g, 2.11 mmol) in THF (15 mL) and water (2 mL) was added NaOH (90 mg, 2.32 mmol), then the reaction mixture was stirred at room temperature for 16 h. Progress of the reaction was monitored by TLC and LCMS. After completion, the reaction mixture was concentrated under vacuum. The residue was extracted with EtOAc (2 × 50 mL). The organic layer was dried over anhydrous Na₂SO₄ and concentrated under vacuum. The residue was purified by column chromatography using 50% EtOAc in hexanes as eluent to afford 300 mg of tert-butyl 4-(4-(furo[3,2-c]pyridin-4-yloxy)-2-(hydroxymethyl)phenyl)-3,5-dimethyl-2,6-dioxo-3,6-dihydropyrimidine-1(2H)-carboxylate **a21** as a white solid.
Yield: 30%
LCMS (ES⁺): 480 (M+H)⁺.
¹H NMR (400 MHz, DMSO-*d₆*) δ 8.14 (s, 1H), 8.01 (d, *J* = 5.87 Hz, 1H), 7.50 (d, J= 5.87 Hz, 1H), 7.40-7.34 (m, 2H), 7.32-7.27 (m, 1H), 7.08 (s, 1H), 5.37 (t, *J* = 5.62 Hz, 1H), 4.36-4.29 (m, 2H), 2.91 (s, 3H), 1.52 (s, 9H), 1.49 (s, 3H).

### D.3.4. Synthesis of tert-butyl 4-(2-(chloromethyl)-4-(furo[3,2-c]pyridin-4-yloxy)phenyl)-3,5-dimethyl-2,6-dioxo-3,6-dihydropyrimidine-1(2H)-carboxylate a22

To a solution of tert-butyl 4-(4-(furo[3,2-c]pyridin-4-yloxy)-2-(hydroxymethyl)phenyl)-3,5-dimethyl-2,6-dioxo-3,6-dihydropyrimidine-1(2H)-carboxylate **a21** (300 mg, 0.63 mmol) in DCM (5 mL) was added a solution of SOCl₂ (0.09 g, 0.75 mmol) DCM (1 mL) at 0 °C, then the reaction mixture was stirred at room temperature for 1 h. Progress of the reaction was monitored by TLC and LCMS. After completion, the reaction mixture was quenched with an aqueous saturated solution of NaHCO₃ (5 mL) and extracted with DCM (2 × 20 mL). The organic layer was dried over anhydrous Na₂SO₄ and concentrated under vacuum. The residue was purified by column chromatography using from 50 to 55% EtOAc in hexanes as eluent to afford 150 mg of tert-butyl 4-(2-(chloromethyl)-4-(furo[3,2-c]pyridin-4-yloxy)phenyl)-3,5-dimethyl-2,6-dioxo-3,6-dihydropyrimidine-1(2H)-carboxylate **a22** as a white solid.
Yield: 48%
HPLC Purity: 98.4%
LCMS (ES⁺): 498 (M+H)⁺.
¹H NMR (400 MHz, DMSO-*d₆*) δ 8.18 (d, *J* = 1.96 Hz, 1H), 8.05 (d, *J* = 5.87 Hz, 1H), 7.57 (d, *J* = 1.96 Hz, 1H), 7.54 (d, *J* = 5.38 Hz, 1H), 7.51-7.48 (m, 1H), 7.46-7.43 (m, 1H), 7.13 (d, *J* = 1.47 Hz, 1H), 4.72 (s, 2H), 2.93 (s, 3H), 1.56 (s, 9H), 1.55 (brs, 3H).

### D.3.5. Synthesis of atropisomer (-) of [¹⁸F]-6-(2-(fluoromethyl)-4-(furo[3,2-c]pyridin-4-yloxy)phenyl)-1,5-dimethylpyrimidine-2,4(1H,3H)-dione (Ib), (-)-(Ib)

In a typical procedure, [¹⁸F]fluoride in a shipping vial (target water obtained from a cyclotron facility) is transferred onto and trapped on an ion exchange cartridge. It is then eluted with a solution of potassium carbonate and Kryptofix 222 into the reaction vessel (RV1) of the TRACERlab® module. The solution is first evaporated by heating at 95°C for 4 min under vacuum and helium flow. Acetonitrile (1 mL) is added to RV1 and the evaporation is continued under the same conditions for 2 min under vacuum. After a second addition of acetonitrile (1 mL), final evaporation is carried out at 95°C for 2 min under vacuum and helium flow. The reactor is then cooled to 50°C.

A solution of the precursor **a22** (0.7 mg) in anhydrous acetonitrile is added to the reaction vessel and the reaction mixture is heated at 80°C for 5 min. After 5 min, the reactor is cooled to 70°C, hydrochloric acid (1M) is added and heated at 70°C for 4 min before being cooled to 40°C and diluted with WFI. The mixture is transferred from RV1 onto an intermediate solid phase extraction cartridge (SPE, Oasis HLB light). RV1 is rinsed with methanol and transferred through SPE to elute the product into RV2, pre-filled with WFI. The entire contents of RV2 are transferred into the HPLC injector loop for purification.

Isolation of **(-)-Ib** is performed by HPLC using a semi-preparative Chiralcel OJ-H column (5 µm, 250 × 10 mm) and Phenomenex Luna C18 (2) (10 µm, 250 x10 mm) and eluted with a mixture of acetonitrile/ammonium acetate solution (5 mM) (40/60, v/v) at a flow rate of 4 mL/min. The product fraction is collected in Flask1, containing 25 mL of ascorbic acid in WFI. The diluted product mixture is passed through a tC18 solid-phase extraction cartridge and the cartridge is rinsed with 10 mL of ascorbic acid in WFI. The radiolabeled product is eluted from the SPE cartridge with 1 mL of 200-proof USP grade ethanol into the formulation flask, pre-loaded with 10 mL of formulation base. The cartridge is rinsed with 4 mL of formulation base and the rinse is mixed with the contents of the formulation flask. The resulting solution is passed through a sterilizing 0.2 µm membrane filter into a sterile, filter-vented vial (final product vial, FPV).

Radiochemical purity was determined using a standard HPLC system, with radioactivity and UV detector connected in series, using analytical Kinetex EVO C18 column (250×4.6 mm, 5 µm) with MeOH/5 mM NH4OAc 50/50, increasing to 80/20 in 15 minutes, as mobile phase, with UV detector set to 254 nm (retention time of (-)-1b = 9.7 minutes, radiochemical purity>99%)

The atropoisomeric purity was determined using a standard HPLC system, with radioactivity and UV detector connected in series, using analytical chiralpak OJ-H column (250x4.6 mm, 5µM), eluted with MeCN/5 mM NH4OAc 40/60 mobile phase at a flow of 1 ml/min, with UV detector set to 254 nm (retention time of (―)-1a = 10.1 minutes, ee>99%).

### D.4. Synthesis of atropisomer (-) of [³H]-6-(2-(fluoromethyl)-4-(furo[3,2-c]pyridin-4-yloxy)phenyl)-1,5-dimethylpyrimidine-2,4(1H,3H)-dione (Ic), (-)-(Ic)

Compound of formula **(―)-(I)** (2mg), Kerr's catalyst (2mg), and dichloromethane (1mL) were combined in a tritiation flask and stirred under 5Ci of tritium gas for 2.5hours. Labile tritium was removed by repeated evaporations to dryness from ethanol. The residues were dissolved in ethanol (20mL). Crude **(-)-(Ic)** was purified by HPLC using a Gemini C18 5µ 250x10mm column, Water + TFA(0.1%) as eluent A, Acetonitrile + TFA (0.1%) as eluent B, 20%B to 100%B over 60min as gradient at 3mL/min flow rate. **(-)-(Ic)** was collected, rotary evaporated to dryness and dissolved in ethanol (20mL).

The radiochemical purity of **(-)-(Ic),** determined by high performance liquid chromatography, was measured using an inertsil ODS3 analytical column (150x4.6 mm, 5µM), eluted with water/TFA as eluent A and CH₃CN/TFA as eluent B phase at a flow of 1 mL/min (gradient ranging from 20% B at t=0 to 100% B at t= 20 minutes) with a radioactivity detector (retention time of **(―)-(Ic)** = 12.9 minutes, radiochemical purity = 99.4%).

LCMS (ES⁺): Main ion at 384 (M+H)⁺ consistent with incorporation of mostly 1 tritium and a specific activity measured at 18Ci/mmol.

### E. BIOLOGY

To assess compound (I) activity on D1 receptor, we tested it in a functional assay. The functional assay measures the stimulation of the production of cyclic adenosine monophosphate (cAMP) in the homogenous time-resolved fluorescence (HTRF) assay, with the maximum increase in cAMP by increasing concentrations of the endogenous agonist, dopamine, defined as 100% of D1 receptor activation. When tested, compound (±)-(I**)** displays significant direct agonist-like effect.

To assess compound (I) affinity and selectivity for D1, it has been tested in competitive radioligand binding assays. Competitive radioligand binding assays were then used to measure (±)-**I** affinity for D1 receptor and assess its selectivity regarding a large panel of 80 related and unrelated targets. Compound (±)-(**I)** binds D1 and D5 receptors with an affinity in the subnanomolar range and do not significantly bind to any of the other tested targets.

Compound **(-)-(I)** has then been tritiated to measure and compare :
1) radioligand binding properties of a known D1 antagonist [³H]SCH23390 and of D1 agonist compound of formula **(-)-(Ic),** and
2) the ability of D1 positive allosteric modulators (PAM) to potentiate agonist or antagonist binding to D1 receptor (D1R). We measured the D1 PAM induced potentiation as an increase in radioligand binding either on cell membrane expressing human D1R either on non-human primate (NHP) brain slices by autoradiography.

The particular assay conditions in which the compound has been tested are described here below.

### E.1. Materials and methods

Transient transfection of human dopamine D1R was performed with pcDNA3.1 vector in human embryonic kidney cells (HEK hD1R).

Brain sections from non-human primate (NHP) Macaca mulatta were provided by Motac (Bordeaux, France) where all animal experiments were performed in accordance with european animal use ethical guidance and legislation .

D1 antagonist compound is [³H]SCH23390 (specific activity of 73 Ci/mmol) which was obtained from Perkin Elmer (Zaventem, Belgium).

HTRF cAMP dynamic assay kit, human neuroepithelioma SK-N-MC cells, and all other reagents were of analytical grade, obtained from conventional commercial sources and used following the manufacturers' recommendations.

Data was analyzed using Excel and Graphpad Prism® softwares. K_{D}, Bmax, pIC₅₀ and pEC₅₀ and Erel were measured by computerized curve fitting according to equations describing the different competitive and allosteric binding and functional activity models.

### E.2. cAMP HTRF D1 functional assay

Dopamine D1R being coupled to Gs-type G protein, its activation triggers an increase in intracellular cAMP concentration ([cAMP]ᵢ). Changes in [cAMP]ᵢ were measured using HTRF technology in human neuroepithelioma SK-N-MC cells endogenously expressing D1R. In 384 well plate, 20,000 cells per well were incubated for 1 hour at room temperature in a final volume of 20 µL Hank's balanced salt solution buffered with 20 mM HEPES (HBSS HEPES buffer, pH 7.4) containing 0.1 mM isobutyl methylxanthine and 10 increasing concentrations of dopamine or of compound of formula (I) (10⁻⁵M to 10⁻¹²M). The reaction was terminated by consecutive additions of 10 µL d2 detection reagent and the cryptate reagent both diluted in lysis buffer. After a period of 60 min at room temperature, changes in HTRF emission ratio were determined and transformed in [cAMP]ᵢ using a standard curve. All incubations were performed in duplicate and results were compared to a concentration-effect curve to dopamine. The potency pEC₅₀ of a compound is the ―log10 of the concentration of the compound which produces 50% of the activation of the cAMP levels and the Erel is the relative efficacy, defined as the maximal % potentiation produced by the compound compared to the maximal response produced by increasing concentrations of dopamine (Erel of 1= dopamine maximum response).

When tested in human neuroblastoma SK-N-MC cells, compound **(-)-I** displayed a D1 partial agonist-like effect with a pEC₅₀ of 8.3 and Erel of 53% as compared to dopamine. Comparative functional results for compound **(-)-1** and dopamine are summarized in Table 1 and further illustrated in Figure 1.

**Table 1: pEC₅₀ and Erel values of (-)-I compound and dopamine for hD1R in SK-N-MC cells**

| Test compound | pEC₅₀ (-logM) | Erel (%) |
|---|---|---|
| Compound (-)-(**I**) | 8.3 ± 0.1 | 53 ± 13 |
| dopamine | 5.8 ± 0.1 | 100 ± 13 |

| | | |
|---|---|---|
| pEC₅₀ and Erel values were obtained from 2 to 4 independent experiments (Average ± SD). | | |

### E.3.Compound (±)-I affinity for human D1R and selectivity profile

Affinity (pKi) of Compound of formula (±)-(**I)** for recombinant human D1R was measured at CEREP (Celle I'Evescault, France) by [³H]SCH23390 radioligand competitive binding experiments. Compound (±)-**I** displayed a pKi of 9.2 for human D1R.

Compound selectivity for human D1R was assessed at 10 µM at CEREP against a panel of 80 targets (n=1 with data in duplicate) including receptors, transporters, enzymes and ion channels. Potentially relevant affinity was identified for only 3 targets: dopamine D5 receptor (D5R), tachykinin NK1 receptors and adenosine transporter (ADET) with >60% inhibition at 10 µM of compound (±)-(**I).** Affinity (pKi) of compound of formula (±)-**I** for human D5R, NK1 receptor and ADET were then measured. Compound of formula (±)-**I** is a D1-like (D1R and D5R) selective ligand (pKi of 9.4 for human D5R), because more than 1000 fold selective with respect to the other 79 tested targets (pKi ≤6.0).

### E.4. Radioligand binding assay

The membrane of HEK hD1R cells were used to measure radioligand binding. 48 hours post transfection, cells were pelleted by centrifugation at 1,500 g and 4°C for 10 min. Pellet was washed once with ice-cold phosphate buffered saline solution (PBS) and centrifuged as described just above. Pellet was homogenized in a buffer containing 15 mM Tris-HCI, 0.3 mM ethylene diamine tetraacetic acid, 1 mM ethylene glycol tetraacetic acid, 2 mM MgCl2 (pH 7.5) and complemented with protease inhibitor cocktail. The homogenate was freeze-thawed twice and equilibrated at 25°C followed by a 10 min DNAse (10U/ml) treatment. Subsequently, the solution was centrifuged at 40,000 g and 4°C for 30 min. Finally, the pellet was resuspended in 20 mM Tris-HCI buffer (pH 7.4) containing 250 mM sucrose and store at -80°C until D1 radioligand binding assay. Membrane preparation of HEK hD1R (5 µg proteins per assay) were incubated for 120 min at 25°C with **(-)-(Ic)** or [³H]SCH23390 in 0.2 ml binding buffer containing 1% DMSO. At the end of the incubation period, the protein-bound radioligand was recovered by filtration through pre-soaked GF/B glass fiber filters. Filters were washed with at least 4 times the assay volume of ice-cold 50 mM Tris-HCI buffer (pH 7.4). The entire filtration step did not exceed 10 sec. The filters were dried and the radioactivity determined by liquid scintillation. Saturation binding assays were carried out using increasing concentrations of **(-)-(Ic)** or [³H]SCH23390 (0.5 to 50 nM) in the absence and in the presence of 10 µM D1 PAMs, Compound a (CAS 1900706-51-5) and Compound b (CAS 1904661-53-5), also referred to as Compound B in paragraph E.7 and in Figures 5 & 6.

Competition/potentiation binding experiments were carried out at constant **(-)-(Ic)** concentration (1 - 4 nM) and 10 increasing concentrations of **(-)-(Ic)** or compound b (10⁻¹⁰M to 10⁻⁵M). In all experiments, the non specific binding (NSB) was defined as the residual radioligand binding observed in the presence of 10 µM of non-labeled respective compound.

D1 PAM compound had no effect on D1 antagonist radioligand [³H]SCH23390 binding (Fig. 3A), but significantly improved binding properties of D1 agonist radioligand **(-)-(Ic)** by increasing Bmax of 45% and decreasing K_{D} of 20% (Fig. 3B).

Ability of D1 PAM compound to potentiate low nanomolar concentration (relevant for a PET tracer) of agonist radioligand (-)-(**Ic)** binding to D1R was demonstrated in figure 3C (>50% potentiation of (-)-**(Ic)** binding).

The resulting curves are set out in Figure 3.

### E.5. Autoradiography

12 µm brain slices were prepared from fresh frozen NHP brain hemisphere and were placed on glass microscopy slide. Autoradiography experiment was performed at room temperature in HBSS HEPES buffer (pH 7.4). NHP brain sections first equilibrated for 30 min before to be incubated for one hour with [³H]compound in 1% DMSO. Slides were then washed in iced-cold 50 mM Tris-HCI buffer (pH 7.4) for 5 min followed by two washes in iced-cold distilled water. Sections were dried under atmospheric pressure at room temperature. Radioactivity on slice was either measured indirectly after exposure of slides to a phosphor screen, either using a direct counter. In every autoradiography experiments, a [³H]standard was included for quantification and the NSB was defined as the residual binding of [³H]compound observed in the presence of 10 µM non-labeled compound.

When tested in autoradiography on NHP brain slices, similarly to known D1-like tracer (see Cadet JL et al. Dopamine D1 receptors, regulation of gene expression in the brain, and neurodegeneration. CNS Neurol Disord Drug Targets. (2010) 9(5): 526―538), (-)-**Ic** demonstrated a significant binding (Total Binding) in caudate putamen consistent with highest levels of D1 receptor expression found in these structures (Cadet et al.). This binding is displaceable and 20% was assessed as non specific binding (measured in the presence of 10 µM compound **(I)**).

In the presence of 10 µM D1 PAM Compound b, (-)-**Ic** specific binding is significantly increased (>150%) showing that there is a significant potentiation.

Figure 4 displays the results obtained in autoradiography assays. Insert shows selected region for data quantification. Data are represented as the mean of duplicates ± standard deviation.

### E.6. In vivo PET imaging studies in Cynomolgus Monkeys

Experiment was performed in a cynomolgus monkey (Female, body weight 6.02 kg). The monkeys were sedated using intramuscular injection of ketamine and kept anesthetized using a mixture of sevoflurane, oxygen and medical air for the duration of the experiment. A baseline scan was performed following intravenous administration of 141 MBq of **(―)-(Ia)** at a specific activity of 795 GBq/µmol and PET images of the brain were acquired using the Siemens HRRT PET scanner (Siemens Preclinical Solutions, Knoxville, TN).

Data were acquired for 123 min and binned into sinograms with the following frame timing: 9 × 20 sec; 3 × 1 min; 5 × 3 min; and 17 × 6 min. Regions of interest used in this study were caudate nucleus, putamen, cerebellum, frontal cortex, occipital cortex, globus pallidus, midbrain, ventral striatum, thalamus and temporal cortex. Registration parameters were obtained to apply the regions of interests to the PET scan and regional time-activity curves (TACs) were generated. Venous blood samples were withdrawn at 4, 15, 30, 60, 90, 120 minutes after (-)-(**Ia)** administration for metabolite analysis.

### Standardized uptake value (SUV) time-activity curves of (-)-la

Standardized uptake value (SUV) time-activity curves of (-)-Ia have been acquired in multiple regions of interest. The peak uptake of (-)-Ia reaches a SUV of about 3.5-4 at 5-10 minutes post-administration across the various brain regions and is followed by a rapid wash-out from the cerebellum to reach a SUV about 1 at 60 minutes as expected from a reference region devoid of D1 receptor.

Wash-out from the caudate and putamen regions of interest is significantly slower to reach a SUVof about 2 at 60 minutes which would be expected from the reported distribution of D1 in those regions. Using the cerebellum as a reference region and the Logan reference tissue model, the binding potential (BP_{ND}) has been calculated respectively as 0.94 and 0.87 in the caudate and putamen which means that compound of formula (-)-(Ia) has a specific binding that can be reliably quantified by PET imaging.

The summed SUV images obtained between 33-63 minutes are demonstrating an uptake pattern consistent with autoradiography in brain tissues and the reported distribution of D1 with high uptake in the striatal structures.

### Metabolites profile of compounds of formula (I)

The profile of metabolites of **(―)-Ia** was determined by radio-HPLC analysis of the venous blood samples collected post-administration at several timepoints. The parent fraction in plasma is 85% at 15 minutes, 60% at 30 minutes and still about 45% at 60 minutes. By contrast with previously reported D1 agonist tracers, only hydrophilic metabolites unlikely to cross the blood-brain barrier have been detected.

### E.7. In vivo potentiation PET imaging studies in Cynomolgus Monkeys

Two cynomolgus monkeys (Macaca fascicularis) housed at the MPI Research (Mattawan, Ml), were used as research subjects for the in vivo potentiation PET studies.

### E.7. 1.Administration of compounds and image acquisitions

Each animal was anesthetized with propofol iv bolus (2.5-5.0 mk/kg) and maintained with propofol iv Continuous Rate Infusion (CRI, 0.1-0.6 mg/kg/min).for at least 30 minutes prior to initiation of Compound-B i.v. dose administration. Several dose levels were administered. For the dose level of 20 mg/kg (total dose), Compound-B was administered as a loading infusion dose of 8.54 mg/kg; administered at a CRI of 34.2 mg/kg/hr over a period of approximately 15 minutes, followed by an infusion maintenance dose of 11.45 mg/kg; to be administered via CRI at a rate of 17.2 mg/kg/hr over a period of approximately 40 minutes. Compound-B administration was discontinued after total infusion time of approximately 55 minutes.Compound **(-)-Ib** was administered iv by bolus injection, with administration initiated 10 minutes post start of Compound-B infusion. Brain imaging data were acquired immediately after initiation of the administration of the radiotracer on the microPET Focus 220 scanner (Siemens Medical Systems, Knoxville, TN). Dynamic emission data were collected in list-mode over 120 min immediately following Compound **(-)-Ib** injection, then reconstructed into a multi frame, dynamic image (6x0.5 min, 3x1 min, 2x2 min, and 22x5 min frames). PET images were corrected for detector normalization, dead time, non-uniform radial sampling, attenuation, scatter, and radioactive decay using software and methodologies provided by PET camera manufacturer.

### E.7.2. Arterial Blood Sampling

Following Compound **(-)-Ib** administration, arterial blood samples were drawn from the central iliac artery prior to the dosing with Compound **(-)-Ib** and at 13 time points up to 120 min post-injection of Compound **(-)-Ib**. All blood samples were collected in anticoagulant tubes and stored on wet ice until quantificationfor radioactivity by gamma counter, measure of Compound **(-)-Ib** parent fraction in plasma over time by HPLC and Compound **(-)-Ib** ex vivo stability in blood.

### E.7.3. Image processing and analysis

Reconstructed dynamic brain PET images were transferred and analyzed using the image processing PMOD software package (PMOD Technologies, Zurich, Switzerland). The PET images acquired at baseline were aligned to previously acquired animal's brain Structural Magnetic Resonance Imaging (MRI). The animal's brain MRI was spatially normalized to a common MR cynomolgus monkey brain template and the resulting normalization transformation was applied to the PET images. Subsequent PET images acquired during drug studies were aligned first to the baseline images and then transformed into the common MR template space using the already calculated transformation from the baseline images.

Regions of interest (ROIs) defined in the template space were applied to the PET images to compute time-activity curves (TACs). Average activity concentration (kBq/cc) within each ROI was determined and TACs representing the regional brain activity concentration over time were generated. Brain TACs and images were presented in SUV units (g/mL) by normalizing by the weight of the animal and the injected dose.

The plasma-based method Logan graphical analysis (LGA) was applied to the regional TACs to determine the total volumes of distribution (V_{T}) for each brain region. Arterial input function used for modeling was generated by correcting the plasma activity for metabolites using the measured parent fraction.

A potentiation plot, similar in concept to the global occupancy Lassen plot, was used where V_{T}^{Treatment} -V_{T}^{Baseline} is on the y-axis and V_{T}^{Treatment} is on the x-axis. The potentiation, defined as the increase in specific signal, potentiation = Vₛ^{Treatment}/V_{T}^{Baseline} where Vs is the specific volume of distribution, can then be derived from the potentiation plot as slope/(1-slope) of the graph, while the non-displaceable distribution volume (V_{ND}), is given by the x-intercept of the graph.

### E.7.4. Results

SUV PET images averaged (time-weighted) over 40-90 min acquired at baseline and post-dosing with Compound-B at 20 mg/kg and associated time-activity curves are shown in Figure 5. Both images show the effect of Compound-B on the uptake of the Compound-B, where an increased uptake is observed due to the potentiation of Compound **(-)-Ib** by the binding of Compound-B to the D1 allosteric site.

To quantify the potentiation effect, the volume of distribution (*V_{T}*) was estimated from the time activity curves at baseline and post-dosing with Compound-B using LGA. At 20 mg/kg, an increase of -100% on average was observed in the caudate nucleus and putamen in a cynomolgus macaque.

The potentiation plot is shown in Figure 6 for the caudate nucleus, putamen, ventral striatum, pallidum, substantia nigra, insula, cingulate and cerebellum.

From the plot, the potentiation was estimated to be approximately 136% for a total dose of Compound-B of 20 mg/kg, infused over 55 min.

## Claims

1. A compound of formula (I), 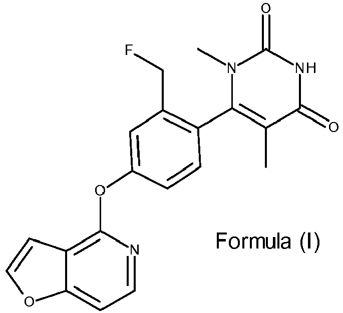 which compound is radiolabeled, or a pharmaceutically acceptable salt thereof.

2. The compound of formula (I) according to Claim 1, or a pharmaceutically acceptable salt thereof wherein at least one of the hydrogen is ³H; or at least one of the carbons is a ¹¹C; or the fluorine atom is a ¹⁸F.

3. The compound of formula (I) according to Claim 1, represented by formula (Ia), or a pharmaceutically acceptable salt thereof, 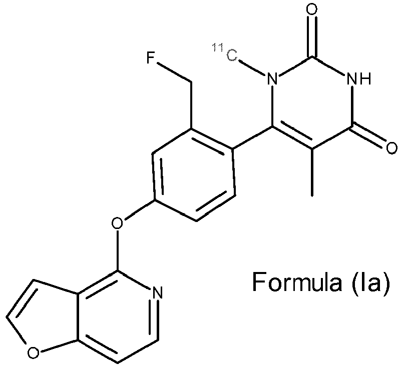 wherein one of the carbon is a ¹¹C.

4. The compound of formula (I) according to Claim 1, represented by formula (lb), or a pharmaceutically acceptable salt thereof, 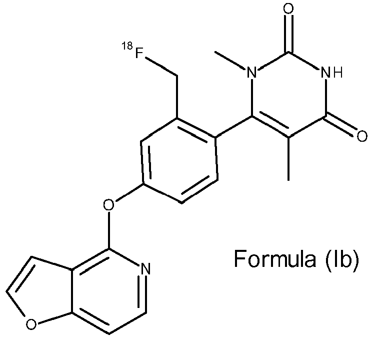 wherein the flurorine is a ¹⁸F.

5. The compound of formula (I) according to Claim 1, represented by formula (Ic), or a pharmaceutically acceptable salt thereof, 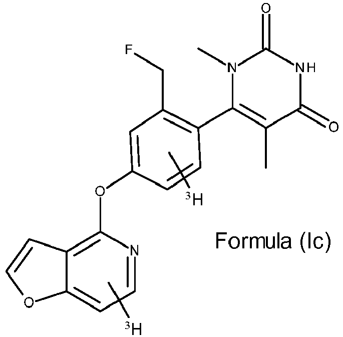 wherein one or more of the hydrogens is ³H.

6. The compound of formula (I) according to any one of the preceding claims which consists essentially of atropisomer (-).

7. A pharmaceutical composition comprising a detectable amount of a compound of formula (I) according to Claim 1, or a pharmaceutically acceptable salt thereof, in combination with a pharmaceutically acceptable diluent or carrier.

8. The compound of formula (I) according to any one of Claims 1 to 6 for use as a radioactive tracer.

9. The compound of formula (I) according to Claim 8 for use in a method of quantification of the density of D1-like receptors.

10. The compound of formula (Ia) according to Claim 3 or the compound of formula (Ib) according to Claim 4, or the pharmaceutical composition according to Claim 7, for use in a method of *in vivo* PET imaging.

11. The Compound of formula (Ia) according to Claim 3 or the compound formula (Ib) according to Claim 4, for use in a method of *in vivo* PET imaging and measuring the target engagement of an orthosteric agonist modulator of D1-like receptors.

12. The Compound of formula (Ia) according to Claim 3 or the compound of formula (Ib) according to Claim 4, for use in the *in vivo* quantification of the effect of D1 allosteric modulators on the D1 receptor.

13. Use of a compound of formula (Ic) according to Claim 5 to measure *in vitro* or *ex vivo* the target engagement of an orthosteric agonist modulator of D1-like receptors.

14. Use of a compound of formula (Ic) according to Claim 5 to quantify *in vitro* or *ex vivo* the effect of D1 allosteric modulators on the D1 receptor.

## Patentansprüche

1. Verbindung der Formel (I) 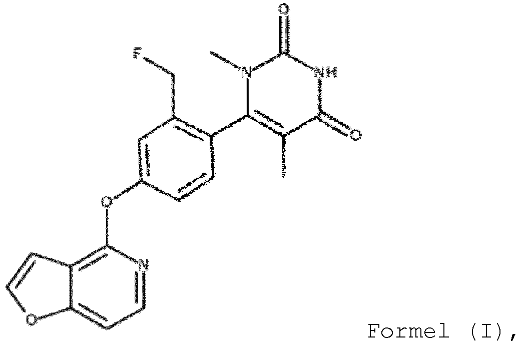 wobei die Verbindung radioaktiv markiert ist, oder pharmazeutisch unbedenkliches Salz davon.

2. Verbindung der Formel (I) nach Anspruch 1 oder pharmazeutisch unbedenkliches Salz davon, wobei mindestens einer der Wasserstoffe ³H ist oder mindestens einer der Kohlenstoffe ein ¹¹C ist oder das Fluoratom ein ¹⁸F ist.

3. Verbindung der Formel (I) nach Anspruch 1, wiedergegeben durch Formel (Ia), oder pharmazeutisch unbedenkliches Salz davon, 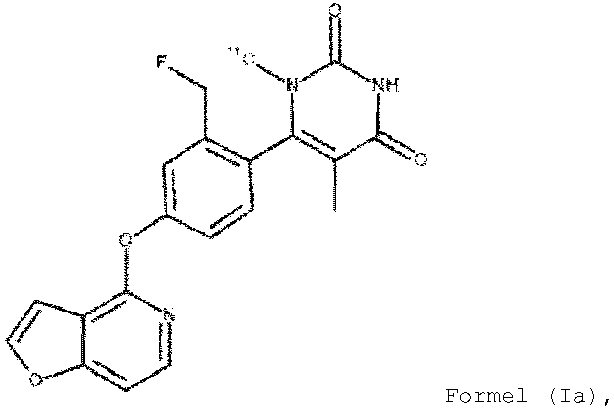 wobei einer der Kohlenstoffe ein ¹¹C ist.

4. Verbindung der Formel (I) nach Anspruch 1, wiedergegeben durch Formel (Ib), oder pharmazeutisch unbedenkliches Salz davon, 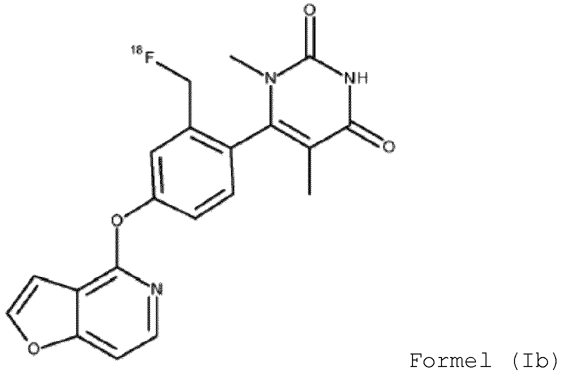 wobei das Fluor ein ¹⁸F ist.

5. Verbindung der Formel (I) nach Anspruch 1, wiedergegeben durch Formel (Ic), oder pharmazeutisch unbedenkliches Salz davon, 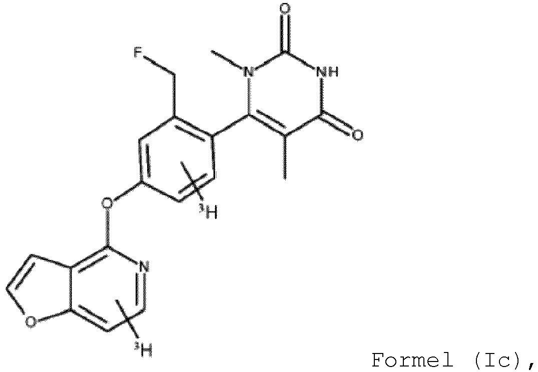 wobei ein oder mehrere der Wasserstoffe ³H sind.

6. Verbindung der Formel (I) nach einem der vorhergehenden Ansprüche, welche im Wesentlichen aus dem Atropisomer (-) besteht.

7. Pharmazeutische Zusammensetzung, umfassend eine nachweisbare Menge einer Verbindung der Formel (I) nach Anspruch 1 oder eines pharmazeutisch unbedenklichen Salzes davon in Kombination mit einem pharmazeutisch unbedenklichen Verdünnungsmittel oder Träger.

8. Verbindung der Formel (I) nach einem der Ansprüche 1 bis 6 zur Verwendung als radioaktiver Tracer.

9. Verbindung der Formel (I) nach Anspruch 8 zur Verwendung in einem Verfahren zur Quantifizierung der Dichte von D1-like-Rezeptoren.

10. Verbindung der Formel (Ia) nach Anspruch 3 oder Verbindung der Formel (Ib) nach Anspruch 4 oder pharmazeutische Zusammensetzung nach Anspruch 7 zur Verwendung in einem Verfahren zur in-vivo-PET-Bilddarstellung.

11. Verbindung der Formel (Ia) nach Anspruch 3 oder Verbindung der Formel (Ib) nach Anspruch 4 zur Verwendung in einem Verfahren zur in-vivo-PET-Bilddarstellung und zum Messen des Target-Engagements eines orthosterischen Agonistenmodulators von D1-like-Rezeptoren.

12. Verbindung der Formel (Ia) nach Anspruch 3 oder Verbindung der Formel (Ib) nach Anspruch 4 zur Verwendung bei der in-vivo-Quantifizierung der Wirkung von D1-allosterischen Modulatoren auf dem D1-Rezeptor.

13. Verwendung einer Verbindung der Formel (Ic) nach Anspruch 5 zum Messen des *in-vitro-* oder *ex*-*vivo*-Target-Engagements eines orthosterischen Agonistenmodulators von D1-like-Rezetoren.

14. Verwendung einer Verbindung der Formel (Ic) nach Anspruch 5 zum *in-vitro-* oder ex-vivo-Quantifizieren der Wirkung von D1-allosterischen Modulatoren auf den D1-Rezeptor.

## Revendications

1. Composé de formule (I), 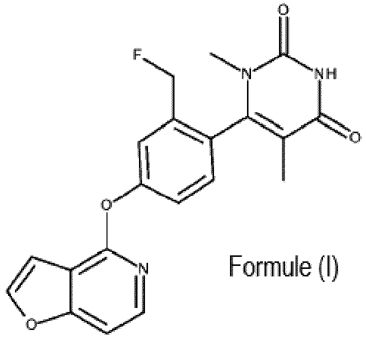 lequel composé étant radiomarqué, ou sel pharmaceutiquement acceptable correspondant.

2. Composé de formule (I) selon la revendication 1, ou sel pharmaceutiquement acceptable correspondant, au moins l'un des hydrogènes étant ³H ; ou au moins l'un des carbones étant un ¹¹C ; ou l'atome de fluor étant un ¹⁸F.

3. Composé de formule (I) selon la revendication 1, représenté par la formule (Ia), ou sel pharmaceutiquement acceptable correspondant, 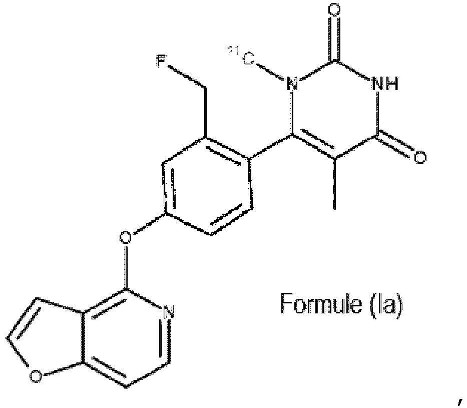 l'un des carbones étant un ¹¹C.

4. Composé de formule (I) selon la revendication 1, représenté par la formule (Ib), ou sel pharmaceutiquement acceptable correspondant, 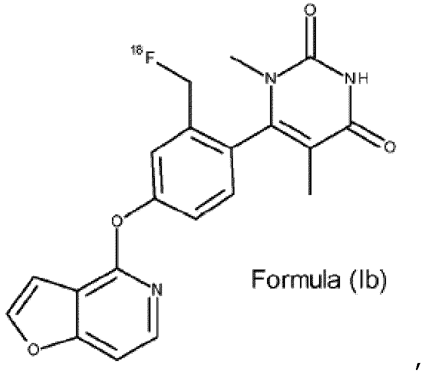 le fluor étant un ¹⁸F.

5. Composé de formule (I) selon la revendication 1, représenté par la formule (Ic), ou sel pharmaceutiquement acceptable correspondant, 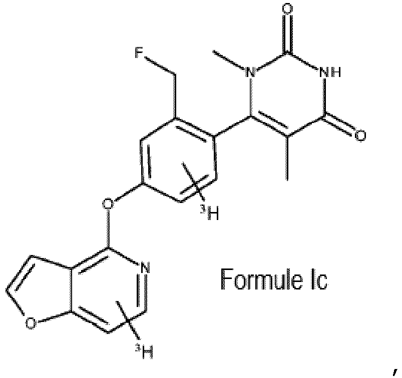 un ou plusieurs des hydrogènes étant ³H.

6. Composé de formule (I) selon l'une quelconque des revendications précédentes qui est constitué essentiellement de l'atropoisomère (-).

7. Composition pharmaceutique comprenant une quantité détectable d'un composé de formule (I) selon la revendication 1, ou d'un sel pharmaceutiquement acceptable correspondant, en combinaison avec un diluant ou support pharmaceutiquement acceptable.

8. Composé de formule (I) selon l'une quelconque des revendications 1 à 6 pour une utilisation en tant que traceur radioactif.

9. Composé de formule (I) selon la revendication 8 pour une utilisation dans un procédé de quantification de la densité de récepteurs D1-like.

10. Composé de formule (Ia) selon la revendication 3 ou composé de formule (Ib) selon la revendication 4, ou composition pharmaceutique selon la revendication 7, pour une utilisation dans un procédé d'imagerie PET *in vivo.*

11. Composé de formule (Ia) selon la revendication 3 ou composé de formule (Ib) selon la revendication 4, pour une utilisation dans un procédé d'imagerie PET *in vivo* et de mesure de l'attaque d'une cible d'un modulateur agoniste orthostérique de récepteurs D1-like.

12. Composé de formule (Ia) selon la revendication 3 ou composé de formule (Ib) selon la revendication 4, pour une utilisation dans la quantification *in vivo* de l'effet de modulateurs allostériques D1 sur le récepteur D1.

13. Utilisation d'un composé de formule (Ic) selon la revendication 5 pour mesurer *in vitro* ou *ex vivo* l'attaque d'une cible d'un modulateur agoniste orthostérique de récepteurs D1-like.

14. Utilisation d'un composé de formule (Ic) selon la revendication 5 pour quantifier *in vitro* ou *ex vivo* l'effet de modulateurs allostériques D1 sur le récepteur D1.
